# EUROPÄISCHE PATENTSCHRIFT

(11) **EP 1 537 142 B1**
(45) Veröffentlichungstag und Bekanntmachung des Hinweises auf die Patenterteilung: **29.06.2011**
(21) Anmeldenummer: 03750490.9
(22) Anmeldetag: 05.09.2003
(51) Int. Cl.: C07K 14/47, C12N 15/12, A61K 38/17, G01N 21/00

(54) **AKAP18 DELTA, EINE NEUE SPLEISSVARIANTE EINES PROTEINKINASE A-ANKERPROTEINS UND VERWENDUNG DIESER**
AKAP18 DELTA, A NOVEL SPLICING VARIANT OF A PROTEIN KINASE A ANCHOR PROTEIN AND THE USE OF THE SAME
NOUVELLE VARIANTE D'EPISSURE AKAP18 DELTA D'UNE PROTEINE D'ANCRAGE DE LA PROTEINE KINASE A ET SON UTILISATION

(30) Priorität: 06.09.2002 DE 10244072; 07.02.2003 DE 10306085
(43) Veröffentlichungstag der Anmeldung: 08.06.2005
(73) Patentinhaber: Forschungsverbund Berlin e.V., 12489 Berlin (DE)
(72) Erfinder: KLUSSMANN, Enno, 12209 Berlin (DE); OKSCHE, Alexander, 12207 Berlin (DE); ROSENTHAL, Walter, 14532 Kleinmachnow (DE)
(74) Vertreter: Hertin, Paul W.
(86) Internationale Anmeldenummer: PCT/EP2003/009892
(87) Internationale Veröffentlichungsnummer: WO 2004/022591

(56) Entgegenhaltungen:
- KLUSSMANN E. ET AL.: "Characterization of a new splice variant of AKAP18: its potential involvement in vasopressin-mediated water reabsorption" NAUNYN-SCHMIEDEBERGS ARCHIVES OF PHARMACOLOGY, Bd. 362, Nr. 4-5 (Supplement), 2000, Seite R28 XP009024324
- KLUSSMANN E. AND ROSENTHAL W.: "Role and Identification of protein kinase A anchoring proteins in vasopressin mediated aquaporin-2 translocation" KIDNEY INTERNATIONAL, Bd. 60, 2001, Seiten 446-449, XP002267810
- TROTTER K.W. ET AL.: "Alternative splicing Regulates the subcellular localization of A-kinase anchoring protein 18 isoforms" THE JOURNAL OF CELL BIOLOGY, Bd. 147, Nr. 7, 27. Dezember 1999 (1999-12-27), Seiten 1481-1492, XP002267811 in der Anmeldung erwähnt
- KLUSSMANN E. ET AL.: "Protein Kinase A anchoring proteins are required for Vasopressin-mediated Translocation of Aquaporin-2 into cell membranes of Renal principal cells" THE JOURNAL OF BIOLOGICAL CHEMISTRY, Bd. 274, Nr. 8, 19. Februar 1999 (1999-02-19), Seiten 4934-4938, XP002267812 in der Anmeldung erwähnt
- OLIVERIA S.F. ET AL.: "Imaging kinase-AKAP79-phosphatase scaffold complexes at the plasma membrane in living cells using FRET microscopy" THE JOURNAL OF CELL BIOLOGY, Bd. 160, Nr. 1, 6. Januar 2003 (2003-01-06), Seiten 101-112, XP002267813

## Beschreibung

Die Erfindung betrifft eine für ein Proteinkinase A-Ankerprotein kodierende Nukleinsäuresequenz, eine Verwendung dieser Nukleinsäuresequenz in einem Fusionsprotein und ein Verfahren zur Bestimmung, der Wechselwirkung des Proteinkinase A-Ankerproteins mit regulatorischen Untereinheiten der Proteinkinase A sowie ein Verfahren zur Identifikation zellpermeabler Substanzen.

Die biologische Wirkung von Hormonen und Neurotransmittern wird über die Aktivierung von Signalkaskaden, welche den Phosphorylierungsstatus von Effektorproteinen verändern, vermittelt. An diesem reversiblen Prozess sind zwei Klassen von Enzymen beteiligt: Proteinkinasen und Phosphoproteinphosphatasen. Die Phosphorylierung erfolgt durch Kinasen, welche die Übertragung der endständigen Phosphatgruppe von ATP auf spezifische Serin- oder Threoninreste katalysieren, die Dephosphorylierung wird durch Phosphoproteinphosphatasen vermittelt. Ein Mechanismus zur Kontrolle und Regulation dieser Enzymaktivitäten ist die Kompartimentierung dieser Enzyme durch die Assoziation mit Ankerproteinen, die in der Nähe ihrer Substrate lokalisiert sind. Die Proteinkinase A (PKA) ist eine der multifunktionellen Kinasen mit einer breiten Substratspezifität, welche durch die so genannten *protein kinase A anchoring proteins* (AKAPs) an subzellulären Strukturen verankert wird.

Bei vielen wichtigen zellulären Prozessen wie Kontraktion, Sekretion, Stoffwechsel, Gentranskription, Zellwachstum und -teilung erfolgt die Weiterleitung extrazellulärer Signale über G-Protein-gekoppelte Rezeptoren, das G-Protein Gₛ, Aktivierung einer Adenylzyklase und Bildung des *second-messenger* zyklischen Adenosinmonophosphats (cAMP). Die Effekte von cAMP werden durch die cAMP-abhängige PKA vermittelt.

Die Untereinheiten der PKA werden beim Menschen von sieben verschiedenen Genen, welche auf unterschiedlichen Chromosomen lokalisiert sind, kodiert. Drei Gene kodieren für die Isoformen der katalytischen Untereinheit Cα, Cβ und Cγ und vier Gene für die Isoformen der regulatorischen Untereinheit RIα, RIß, RIIα und RIIß.

Die regulatorischen Untereinheiten zeigen ein unterschiedliches Expressionsmuster. Während RIα und RIIα ubiquitär in den Geweben vorkommen, ist die regulatorische Untereinheit RIß in erster Linie im Gehirn zu finden.

Die Assoziation der RII-Untereinheiten mit intrazellulären Kompartimenten wird durch AKAPs vermittelt. Bei den Ankerproteinen handelt es sich um eine Gruppe funktionell verwandter Moleküle, die durch die Interaktion mit Typ I bzw. Typ II der regulatorischen Untereinheiten (RI bzw. RII) des PKA-Holoenzyms charakterisiert sind. Die ersten Ankerproteine wurden bei der affinitätschromatographischen Reinigung der R-Untereinheiten über cAMP-Sepharose isoliert. Diese assoziierten Proteine zeigten auch nach Transfer auf eine Nitrozellulosemembran eine RII-Bindung. Auf dieser Beobachtung beruht auch die bisherige Methode (RII-overlay) zur Detektion von AKAPs. Es handelt sich hierbei um einen modifizierten Western Blot, bei dem statt eines primären Antikörpers radioaktiv markierte RII-Untereinheiten als Sonde eingesetzt werden.

Zur funktionellen Bedeutung der RI-AKAP-Interaktion ist noch wenig bekannt. Auch wenn RIα hauptsächlich zytosolisch lokalisiert ist, zeigen verschiedene Studien eine Verankerung *in vivo.* Dabei scheint die dynamische Verankerung der RIα-Untereinheiten im Gegensatz zur statischen Verankerung der RII-Untereinheiten von entscheidender Bedeutung für die Zelle zu sein. So wurde die Assoziation der RI-Untereinheiten mit der Plasmamembran von Erythrozyten und aktivierten T-Lymphozyten beschrieben. Bei der cAMP-vermittelten Inhibition der T-Zell-Proliferation durch die PKA Typ I könnte die Lokalisation des Enzyms möglicherweise auch durch AKAPs vermittelt werden. In *knockout*-Mäusen, die im Skelettmuskelgewebe keine regulatorischen Untereinheiten Typ II exprimieren, binden die RIa-Untereinheiten an ein mit Kalziumkanälen assoziiertes AKAP und erhalten so die normale, cAMP-abhängige Kanalleitfähigkeit durch die korrekte Verfügbarkeit der katalytischen Untereinheiten der PKA.

*In vivo* konnte Weiterhin gezeigt werden, dass die katalytischen Untereinheiten in der Zelle bevorzugt mit den RII-Untereinheiten assoziieren und Typ I-PKA-Holoenzyrn gebildet wird, wenn die Menge der freien katalytischen Untereinheiten die Menge der freien RII-Untereinheiten übersteigt.

Die Spezifität in der PKA-Verankerung wird durch die *targeting*-Domäne erreicht, ein Strukturmotiv, das im Gegensatz zu der *anchoring*-Domäne weder in der Sequenz noch in der Struktur der AKAPs konserviert ist. So werden AKAPs durch Protein-Protein-Interaktionen an strukturelle Elemente in der Zelle und durch Protein-Lipid-Interaktionen an Membranen verankert.

In der Literatur sind verschiedene AKAPs beschrieben, die mit unterschiedlichen zellulären Kompartimenten assoziieren, so zum Beispiel mit den Zentrosomen, den Mitochondrien, dem endoplasmatischen Retikulum und dem Golgi-Apparat, der Plasma- und Kernmembran und mit Vesikeln.

Die genauen Mechanismen der Verankerung sind bisher nur für einige AKAPs bekannt. So wird das herzmuskelspezifische Ankerprotein mAKAP durch eine Region mit drei spektrinartigen Wiederholungssequenzen an der perinukleären Membran der Kardiomyozyten verankert. Zwei Isoformen der AKAP15/18 werden durch Lipidmodifikationen (Myristoylierung und Palmitoylierung) an der Plasmamembran verankert. Drei polybasische Regionen in der targeting-Domäne des AKAP79 sind an der Lokalisation des Proteins an der inneren postsynaptischen Membran (PSD, *postsynaptic density*) beteiligt.

Die AKAPs wurden zuerst durch die Interaktion mit der PKA charakterisiert., Einige dieser Proteine können jedoch auch andere an der Signaltransduktion beteiligte Enzyme binden. Durch die gleichzeitige Verankerung von Enzymen, die gegensätzliche Reaktionen katalysieren, wie zum Beispiel Kinasen und Phosphatasen, können diese, auch als *scaffolding* (gerüstbildende) Proteine bezeichneten AKAPs ganze Signalkomplexe in der Nähe bestimmter Substrate lokalisieren und so zur Spezifität und Regulation der zellulären Antwort auf extrazelluläre Signale beitragen. AKAP79 war das erste AKAP, für das die Interaktion mit mehreren Enzymen nachgewiesen werden konnte. Dieses Protein bindet die Proteinkinase A; die Proteinkinase C und die Proteinphosphatase Calcineurin (PP2B), wobei jedes Enzym in gebundenem Zustand inhibiert ist. Da unterschiedliche Signale für die Aktivierung jedes einzelnen Enzyms notwendig sind, können an dieser Stelle verschiedene *second messenger* wie cAMP, Kalzium und Phospholipide zusammentreffen. Weitere Beispiele sind das AKAP220, welches die PKA und die Proteinphosphatase PP1 an den Peroxisomen lokalisiert und das AKAP Yotiao, das neben der PKA ebenfalls die Proteinphosphatase PP1 bindet. Das AKAP CG-NAP bindet nicht nur die PKA und die Proteinphosphatase PP1, sondern auch noch die Rho-abhängige Kinase PKA (NGF (nerve growth factor) -aktivierte Proteinkinase) und die Proteinphosphatase PP2A.

Im Stand der Technik ist ein AKAP 18γ bekannt, was in verschiedenen Geweben wie in Wasserkanälen oder im Herzen entweder nicht exprimiert vorliegt oder aber keine Funktion wahrnimmt. Mit den bekannten AKAPs ist es auch noch nicht gelungen die Interaktion, beispielsweise mit PKA, zu visualisieren. Es ist lediglich durch Publikationen wie z. B. Klussmann et al. (1999) bekannt, dass auf Grund von gewissen Ergebnissen von einer Wechselwirkung ausgegangen werden muss, ohne dass diese selbst analysiert werden konnte.

Die folgende Druckschriften offenbaren weitere AKAP-Molekülen und gehören ebenfalls zum Stand der Technik: Klussmann et al., Archives of Pharmacology (2000), Klussmann und Rosenthal, Kidney International (2001), Trotter et al., Journal of Cell Biology (1999) and Oliveria et al., Journal of Cell Biology (2003). In diesen Dokumenten sind allerdings weder die erfindungsgemäßen Nukleinsäuremoleküle, noch relevante oder naheliegende Alternativen der vorliegenden Erfindung offenbart.

Auch andere Proteine können mit AKAPs assoziierten, so bindet Ezrin, ein Mitglied der zytoskelett-assoziierten ERM-Familie Ezrin, Radixin und Moesin, das als AKAP identifiziert wurde, an ein Protein (EBP50/NHERF), welches an der Regulation des Natrium-Protonen-Transportes in der apikalen Membran von Epithelzellen beteiligt ist. AKAPs vermitteln die Modulation der Leitfähigkeit der Ionenkanäle durch die Lokalisation der Proteinkinasen und -phosphatasen in der Nähe bestimmter Kanaluntereinheiten, die wahrscheinlich durch Phosphorylierung und Dephosphorylierung reguliert werden.

Die Aktivität des NMDA-Rezeptors wird durch das AKAP Yotiao, welches auch die Proteinphosphatase PP1 bindet, moduliert. Die in gebundenem Zustand aktive Phosphatase limitiert die Kanalleitfähigkeit des NMDA-Rezeptors, bis die PKA durch cAMP aktiviert wird und den Ionenkanal oder ein assoziiertes Protein phosphoryliert, wodurch die Leitfähigkeit rapide ansteigt. Es konnte weiterhin gezeigt werden, dass myristoylierte Ht31-Peptide, die die Interaktion zwischen PKA und AKAP inhibieren, die cAMP-abhängige Inhibition der Interleukin 2-Transkription in Jurkat-T-Zellen aufheben und dass S-Ht31-Peptide die Spermienmotilität einschränken.

Auch bei den wichtigen komplexen biologischen Prozessen, wie die durch das Hormon GLP-1 (*glucagon-like peptide*)-vermittelte Insulinsekretion in den β-Zellen des Pankreas und in RINm5F-Zellen (klonale β-Zelllinie der Ratte) sind AKAPs beteiligt. Die Aktivierung der PKA durch GLP-1 führt zur Phosphorylierung von L-Typ-Kalziumkanälen und begünstigt die Exozytose von Insulin aus sekretorischen Granula. Die Ht31-Peptid-vermittelte Inhibition der PKA-Verankerung führte zu einer deutlichen Verringerung der Insulinsekretion. Dabei wurden weder die cAMP-Bildung noch die Aktivität der katalytischen Untereinheiten der PKA durch die Peptide beeinflusst. Weiterhin konnte nach Expression des wildtypischen AKAP18α in RINm5F-Zellen im Vergleich zu Kontrollzellen, welche AKAP18α nicht exprimierten, eine Erhöhung der Insulinsektretion nach GLP-1-Applikation nachgewiesen werden.

Bisher wurde der Nachweis, dass es sich bei einem neuen Protein um ein AKAP handelt, durch Kopräzipitation erbracht. Dazu würde ein Antikörper gegen das Kandidatenprotein hergestellt, um es aus Zellen oder Gewebe, in denen es exprimiert wird, immunzupräzipitieren. Anschließend wurde das Vorhandensein von regulatorischen und/oder katalytischen PKA-Untereinheiten im Präzipitat mittels Western Blot untersucht. Die Anwesenheit der PKA-Untereinheiten im Präzipitat spricht dafür, dass das Kandidatenprotein in vivo als AKAP fungiert. Der Nachweis kann auch umgekehrt erbracht werden, indem die PKA-Untereinheiten immunpräzipitiert werden und anschließend das AKAP im Präzipitat nachgewiesen wird.

Dieser experimentelle Ansatz erlaubt aber keinen Rückschluss auf die intrazelluläre Lokalisation oder auf die in vivo-Situation eines AKAP-PKA-Komplexes. Es ist außerdem nicht möglich, die zeitliche und räumliche Auflösung von Interaktionen zu analysieren. Weiterhin ist es derzeit mit den bekannten Verfahren nicht möglich, spezifische AKAP-Inhibitoren oder -Aktivatoren zu identifizieren.

Aufgabe der Erfindung ist es daher, neue Nukleinsäuresequenzen, die für Strukturen kodieren, zur Verfügung zu stellen, die in Verfahren eingesetzt werden können, in denen die Interaktion zwischen AKAP und PKA in vivo detektiert wird, wobei die Rückschlüsse auf die zelluläre Lokalisation dieser Interaktion erhalten werden können und die weiterhin in Verfahren eingesetzt werden können, mit denen membranpermeable Substanzen, insbesondere Peptide, detektierbar sind.

Die vorliegende Erfindung löst dieses technische Problem durch die Bereitstellung einer isolierten Nukleinsäuresequenz ausgewählt aus der Gruppe umfassend:
a) ein Nukleinsäuremolekül umfassend eine Nukleotidsequenz ausgewählt aus der Gruppe bestehend aus SEQ ID Nr. 1 oder deren komplementären Nukleotidsequenzen,
b) ein Nukleinsäuremolekül, welches mit einer Nukleotidsequenz gemäß a) unter stringenten Bedingungen hybridisiert, und für ein Protein kodiert, welches mit AQP2 on intra Zellulären Vesikeln in renalen Hauptzellen lokalisierbar ist;
c) ein Nukleinsäuremolekül, das in Folge des genetisches Codes zu einer Nukleotidsequenz gemäß a) degeneriert ist.

Die Bedingungen der Hybridisierung sind hierbei so gewählt, dass eine Hybridisierung mit bekannten Nuckleinsäuremolekülen, insbesondere solchen, die für bekannte Spleißvarianten codieren, ausgeschlossen sind.

Es war überraschend, dass die erfindungsgemäßen Nukleinsäuresequenzen eingesetzt werden können, um die Interaktion von AKAP und PKA Untereinheiten in vivo zu detektieren, wobei ein AKAP-PKA-Komplex einem zellulären Kompartiment zugeordnet werden kann, wobei sich der Begriff AKAP in diesem Zusammenhang auf die neue Spleißvariante bezieht.

Im Sinne der Erfindung heißt, um zu den genannten Nukleinsäuresequenzen bzw. den mit diesen Nukleinsäuresequenzen hybridisierenden Sequenzen funktionsanalog zu sein, dass dies kodierten homologen Strukturen bei der Interaktion mit PKA-Untereinheiten Merkmale aufweisen, die Rückschlüsse auf die in vivo Situation und die zelluläre Lokalisation zulassen sowie auf die Identifizierung spezifischer AKAP-Inhibitoren. Funktionsanalog heißt insbesondere, dass die kodierten Moleküle ein analoges Verhalten zu der neuen Spleißvariante AKAP 18 δ zeigen.

Mit dieser neuen Spleißvariante lassen sich Aufgaben lösen, die beispielsweise mit der bekannten Spleißvariante AKAP18γ nicht zu lösen waren. Die Unterschiede zwischen dem bekannten AKAP18γ und dem erfindungsgemäßen AKAP18δ betragen auf der Nukleinsäureebene ca. 20%.

Die Proteinkinase A-Ankerproteine AKAPγ und das erfindungsgemäße AKAP18δ sind verschiedene Spleißvarianten des AKAP18-Gens. AKAP18δ enthält im Vergleich zu AKAP18γ 27 zusätzliche Aminosäuren am N-Terminus, die durch ein Exon kodiert werden. AKAPs18δ besteht aus 353 und AKAP18γ aus 226 Aminosäuren. Im überlappenden Sequenzbereich besteht eine 75 %ige Identität zwischen den beiden Proteinen.

Diese Unterschiede führen zu deutlichen Unterschiede in der Funktion zwischen dem bekannten und dem neuen Molekül. So ist z. B. AKAPδ im Gegensatz zu AKAP18γ so an wasserkanalhaltigen Vesikeln positioniert, dass es eine essentielle Funktion wahrnehmen kann. Weiterhin ist AKAPδ an den Ca²⁺-Kanälen im Herzen lokalisiert und nimmt dort wichtige Funktionen wahr, wohingegen AKAP18γ im Herzen überhaupt nicht exprimiert wird. Demgemäß bestehen deutliche Unterschiede zwischen der bekannten und der anmeldungsgemäßen neuen Spleißvariante von AKAP18. Die neue Spleißvarianten führt zu neuen und überraschenden Ergebnissen. So kann beispielsweise mit AKAP18δ die Interaktion mit PKA besonders gut und effektiv visualisiert werden. Auch wenn es zwischen dem bekannten AKAP18γ und dem erfindungsgemäßen AKAP18δ strukturelle Ähnlichkeiten gibt, sind die neuen Moleküle als Mittel zur erfindungsgemäßen Lösung besser geeignet als die bekannten. Ein Fachmann hätte weder auf Grund des allgemeinen Fachwissens oder auf Grund einer bestimmten Offenbarung annehmen können, dass die bestehenden strukturellen Unterschiede dieser beiden Moleküle so gering sind, dass sie keinen wesentlichen Einfluss auf die Eigenschaften haben, die für die Lösung der technischen Aufgabe von Bedeutung sind, und deshalb vernachlässigt werden können. Es ist aber vielmehr so, dass zwischen AKAP18δ und AKAP18γ neben den Gemeinsamkeiten deutliche strukturelle, wie auch funktionelle Unterschiede bestehen. Diese zeigen sich beispielsweise darin, dass AKAP18δ in bestimmten Regionen des Körpers, wie z. B. in den Ca²⁺-Kanälen des Herzens exprimiert wird, wohingegen die bekannte Verbindung hier nicht exprimiert vorliegt.

In einer weiteren vorteilhaften Ausführungsform der Erfindung weist das Nukleinsäuremolekül mindestens 80 %, bevorzugt 90 % Homologie zu dem erfindungsgemäßen Nukleinsäuremolekül auf.

In einer weiteren bevorzugten Ausführungsform der Erfindung ist das Nukleinsäuremolekül eine genomische DNA und/oder eine RNA; besonders bevorzugt ist das Nukleinsäuremolekül eine cDNA.

Die Erfindung betrifft auch einen Vektor, der mindestens ein erfindungsgemäßes Nukleinsäuremolekül umfasst. Weiterhin betrifft die Erfindung auch eine Wirtszelle, die den Vektor umfasst. Die Erfindung betrifft ganz besonders bevorzugt auch ein Polypeptid, was durch ein erfindungsgemäßes Nukleinsäuremolekül kodiert wird. Ein solches Polypeptid ist bevorzugt eine neue Spleißvariante des AKAP18 (AKAP18δ).

Die Erfindung betrifft auch das Polypeptid bzw. Protein, das durch das erfindungsgemäße Nukleinsäuremolekül kodiert wird.

Die Erfindung betrifft auch einen Antikörper. Die Antikörper im Sinne der Erfindung binden die erfindungsgemäßen Polypeptide, insbesondere AKAP18δ, spezifisch. Die Antikörper können auch modifizierte Antikörper sein (z. B. oligomere, reduzierte, oxidierte und markierte Antikörper). Der in der vorliegenden Beschreibung verwendete Begriff Antikörper umfasst sowohl intakte Moleküle als auch Antikörper-Fragmente, wie Fab, F(ab')₂ und Fv, die bestimmte Epitop-Determinanten der Polypeptide binden können. Bei diesen Fragmenten ist die Fähigkeit des Antikörpers zur selektiven Bindung seines Antigens oder Rezeptors teilweise erhalten geblieben, wobei die Fragmente wie folgt definiert sind:
(1) Fab, das Fragment, das ein monovalentes Antigenbindungsfragment eines Antikörper-Moleküls enthält, lässt sich mittels Spaltung eines ganzen Antikörpers mit dem Enzym Papain erzeugen, wobei eine intakte leichte Kette und ein Teil einer schweren Kette erhalten werden;
(2) das Fab'-Fragment eines Antikörper-Moleküls lässt sich mittels Behandlung eines ganzen Antikörpers mit Pepsin und anschließender Reduktion gewinnen, wobei eine intakte leichte Kette und ein Teil der schweren Kette erhalten werden; pro Antikörper-Molekül werden zwei Fab'-Fragmente erhalten;
(3) F(ab')₂, das Fragment des Antikörpers, das sich mittels Behandlung eines ganzen Antikörpers mit dem Enzym Pepsin ohne anschließende Reduktion erhalten lässt; F(ab')₂ ist ein Dimer von zwei Fab'-Fragmenten, die durch zwei Disulfid-Bindungen zusammengehalten werden;
(4) Fv, definiert als gentechnisch verändertes Fragment, das den variablen Bereich der leichten Kette und den variablen Bereich der schweren Kette enthält und in Form von zwei Ketten exprimiert wird; und
(5) Einzelketten-Antikörper ("SCA"), definiert als gentechnisch verändertes Molekül, das den variablen Bereich der leichten Kette und den variablen Bereich der schweren Kette enthält, die durch einen geeigneten Polypeptid-Linker zu einem genetisch fusionierten Einzelketten-Molekül verbunden sind.

Der in der vorliegenden Erfindung verwendete Begriff Epitop bedeutet eine beliebige Antigen-Determinante auf dem Polypeptid, insbesondere AKAP18δ; Epitop-Determinanten bestehen normalerweise aus chemisch aktiven Oberflächen-Gruppierungen von Molekülen, wie Aminosäuren oder Zucker-Seitenketten, und besitzen normalerweise sowohl spezifische Merkmale der dreidimensionalen Struktur als auch spezifische Ladungsmerkmale.

Die Erfindung betrifft auch Vakzine oder eine pharmazeutische Zusammensetzung, die das Nukleinsäuremolekül, den Vektor, die Wirtszelle, das Polypeptid und/oder das Erkennungsmolekül gegebenenfalls mit einem pharmazeutisch verträglichen Träger umfassen. Bei dem pharmazeutisch akzeptablen Träger handelt es sich um an sich bekannte pharmazeutische Hilfs- und/oder Zusatzstoffe. Bei diesen, dem Fachmann an sich bekannten Zusatz- und Trägerstoffen, kann es sich auch um Liposomen bzw. um in der Gentechnik bekannte Strukturen bzw. Lösungen und/oder Puffergemische oder um andere Substanzen aus dem Bereich der Galenik handeln.

Weiterhin betrifft die Erfindung einen Kit, der die Nukleinsäuren, die Vektoren, die Wirtszelle, das Polypeptid, das Erkennungsmolekül und/oder die pharmazeutische Zusammensetzung umfasst. Der Kit kann z. B. als Diagnosekit oder als Detektionskit verwendet werden, um insbesondere AKAP-Inhibitoren oder die AKAP-PKA-Interaktion zu detektieren.

Die Erfindung betrifft auch ein Verfahren zur Detektion einer AKAP-PKA-Interaktion umfassend die Schritte
a) Bereitstellung eines ersten Vektors, insbesondere eines Plasmids, umfassend ein erfindungsgemäßes Nukleinsäuremolekül kodierend (i) ein AKAP, insbesondere ein AKAP18δ, und (ii) einen ersten Marker, insbesondere ein fluoreszierendes Protein,
b) Bereitstellung eines zweiten Vektors, insbesondere eines Plasmids, umfassend ein zweites Nukleinsäuremolekül kodierend (i) eine regulatorische Untereinheit einer Proteinkinase, bevorzugt RIIα, RIIß, RIα oder RIß, und (ii) einen zweiten Marker, insbesondere ein fluoreszierendes Protein,
c) Einführen des ersten und zweiten Vektors in eine Zelle, wobei die Zelle transfiziert wird, und
d) Durchführen einer Fluoreszenzresonanzenergietransfer-(FRET)-Messung, wobei die AKAP-PKA-Interaktion detektiert wird.

Mit dem erfindungsgemäßen Verfahren ist es überraschenderweise möglich, die AKAP-PKA-Interaktion, bevorzugt eine AKAP18-PKA-, besonders bevorzugt eine AKAP18δ-PKA-Interaktion, in einer lebenden Zelle zu visualisieren und somit den AKAP-PKA-Komplex einem zellulären Kompartiment zuzuordnen.

In einem ersten Schritt des erfindungsgemäßen Verfahrens werden zwei Vektoren bzw. Plasmide bereitgestellt, wobei z. B. das erste Plasmid das erfindungsgemäße Nukleinsäuremolekül, welches für AKAP18δ kodiert, und mindestens ein weiteres Nukleinsäuremolekül umfasst, das für einen Marker, vorzugsweise für ein fluoreszierendes Protein kodiert. Das zweite Plasmid umfasst ebenfalls mindestens zwei Nukleinsäuremoleküle, wobei ein erstes Nukleinsäuremolekül die regulatorische Untereinheit einer Proteinkinase, vorzugsweise RIIα, kodiert und ein weiteres Nukleinsäuremolekül einen zweiten Marker, insbesondere ein zweites fluoreszierendes Fluoreszenzprotein, kodiert. Das erste und das zweite fluoreszierende Protein können hierbei insbesondere so ausgewählt werden, dass sie bei einer ausreichenden räumlichen Nähe zueinander zu einem Fluoreszenzresonanzenergietransfer (FRET) befähigt sind. Demgemäß kann es sich bei dem ersten fluoreszierenden Protein beispielsweise um das Cyan-fluoreszierende Protein (CFP) und bei dem zweiten fluoreszierenden Protein um das Yellow-fluoreszierende Protein (YFP) handeln. Dem Fachmann ist selbstverständlich bekannt, dass er vielfältige Moleküle verwenden kann, um eine messbare Interaktion zwischen Fluoreszenzmarkern wie z. B. einen Fluoreszenzresonanzenergietransfer zu ermöglichen oder einen bestehenden Fluoreszenzresonanzenergietransfer so zu modifizieren, beispielsweise zu inhibieren, dass eine Detektion einer Wechselwirkung von mindestens zwei Markermolekülen, insbesondere Fluoreszenzmarkern, möglich ist. Hierzu ist es erforderlich, dass der erste und zweite Vektor, die insbesondere Plasmide sind, mindestens eine Struktur aufweisen, die messbar als Markierung detektiert werden kann. Der Begriff Marker oder Markierung betrifft im Sinne der Erfindung alle Strukturen oder Verfahren, die zur Erzeugung eines nachweisbaren, vorzugsweise quantifizierbaren Signals verwendet werden können, und die insbesondere an eine Nukleinsäure oder ein Protein bzw. ein Fragment hiervon gebunden oder wirkverbindbar sind. Die Marker oder die Markierungen können insbesondere mittels Fluoreszenz nachweisbare Signale erzeugen. Im Zusammenhang mit der erfindungsgemäßen Lehre wird die Wechselwirkung bzw. eine Modifikation der Wechselwirkung - z. B. als Inhibierung - bevorzugt in Form einer FRET-Messung detektiert. Selbstverständlich ist es auch möglich, mit Hilfe von Radioaktivität, Kolorimetrie, Gravimetrie, Röntgenbeugung oder -absorption, Magnetismus oder enzymatischer Aktivität Signale zu erzeugen, die isoliert gemessen oder im Zusammenhang mit einer Fluoreszenz bzw. einem Fluoreszenzresonanzergietransfervorgang Signale erzeugen, inhibieren oder modifizieren, so dass eine Interaktion von mindestens zwei biologischen Komponenten, vorzugsweise von zwei Proteinen, besonders bevorzugt zwischen einer Proteinkinase, bevorzugt PKA, und einem Proteinkinase A-Ankerprotein, bevorzugt AKAP18δ, nachgewiesen werden kann. Eine Sonde im Sinne der Erfindung ist z. B. eine Nukleinsäure oder Aminosäuresequenz, die an einem oder beiden Enden oder intern mindestens eine Markierung aufweist, wobei die Markierung bevorzugt ein zur Fluoreszenz befähigter Farbstoff oder Marker oder ein die Fluoreszenz unterdrückender Farbstoff oder Marker ist. Eine Sonde im Sinne der Erfindung kann daher aber auch eine Nukleinsäure- oder Aminosäuresequenz sein, die mindestens eine Markierung aufweist, die in der Lage ist, ein nachweisbares Signal zu modifizieren, insbesondere zu inhibieren. Eine solche Sonde kann beispielsweise eine Quencherstruktur sein, die die Fluoreszenz beispielsweise eines Markers oder Farbstoffs, z. B. eines Reporterfarbstoffs, im Zusammenhang mit einer Interaktion zwischen zwei Molekülen so beeinflusst, dass eine messbare Signaländerung erzeugt werden kann. So kann beispielsweise diese Quencherstruktur so ausgebildet sein, dass durch die Wechselwirkung mit einem Fluoreszenzfarbstoff kein Fluoreszenzsignal oder aber kein Energietransfersignal detektierbar ist, wenn die zur Fluoreszenz befähigte Struktur und die zur Quenchung befähigte Struktur die hierfür erforderliche räumliche Nähe, beispielsweise bei einer Interaktion von Proteinase A-Ankerproteinen und Proteinkinasen aufweisen; in einem solchen System wäre ein Fluoreszenzsignal bzw. ein nicht-modifiziertes Fluoreszenzsignal so lange detektierbar, wie es zu einer Interaktion zwischen den markierten Strukturen kommt. Die Begriffe Quenching, Fluoreszenzresonanzenergietransfersignal oder einfach Fluoreszenz betreffen demgemäß Strukturen und Verfahren, durch die, falls ein fluoreszierendes und ein anderes fluoreszierendes bzw. ein quenchendes Molekül räumlich nahe benachbart liegen, bei einer Anregung eines dieser Moleküle ein wesentlicher Teil der Energie des angeregten Zustandes ohne Strahlung auf den Quencher übertragen wird oder mit einer messbaren Strahlung an das System abgegeben wird. Auf den Quencher übertragene Energie kann beispielsweise ohne Strahlung verloren gehen oder in einer anderen Emissionswellenlänge als die des fluoreszierenden Moleküls emittiert werden. Das heißt, die Wechselwirkung zwischen zwei Proteinen oder den sie kodierenden Nukleinsäuren, insbesondere solchen, die mit der Proteinkinase A und dem Proteinkinase A-Ankerprotein assoziiert sind, kann entweder durch eine emittierende Strahlung oder aber durch die strahlungslose Übertragung der Energie auf einen Quencher detektiert werden.

Eine praktische Anleitung für die Auswahl geeigneter Fluoreszenz-Quencher-Paare für bestimmte Sonden ist in der Literatur verfügbar und in den nachstehenden Referenzen beispielhaft dargestellt: Pesce et al., Hrsg., Fluorescence Spectroscopy (Marcel Dekker, New York, 1971), White et al., Fluorescence Analysis: A Practical Approach (Marcel Dekker, New York, 1970). Die Literatur enthält auch Referenzen, die ausführliche Listen von fluoreszenten und chromogenen Molekülen und deren relevante optische Eigenschaften für die Auswahl von Fluoreszenz-Quencher-Paaren bereitstellen; vgl. z.B. Berlman, Handbook of Fluorescence Spectra of Aromatic Molecules, 2. Auflage (Academic Press, New York, 1971), Griffiths, Colour and Constitution of Organic Molecules (Academic Press, New York, 1976), Bishop, Hrsg., Indicators (Pergamon Press, Oxford, 1972), Haugland, Handbook of Fluorescent Probes and Research Chemicals (Molecular Probes, Eugene, 1992). Ferner findet sich in der Literatur eine ausführliche Anleitung für die Derivatisierung von Fluoreszenz- und Quencher-Molekülen für die kovalente Bindung über gewöhnliche reaktive Gruppen, die an ein Oligonukleotid angefügt werden können; vgl. US-PS 3,996,345, US-PS 4,351,760.

Beispielhafte Fluoreszenz-Quencher-Paare können aus Xanthen-Farbstoffen, einschließlich Fluoresceinen und Rhodamin-Farbstoffen ausgewählt werden. Viele geeignete Formen dieser Verbindungen sind kommerziell erhältlich und enthalten Substituenten auf ihren Phenylgruppen, die als Bindestelle oder als Bindefunktionalität für die Bindung an ein Oligonukleotid verwendet werden können. Eine weitere Gruppe von fluoreszenten Verbindungen sind Naphthylamine mit einer Aminogruppe in der alpha- oder beta-Position. Diese Naphthylamino-Verbindugen umfassen 1-Dimethylaminonaphthyl-5-sulfonat, 1-Anilino-8-naphthalensulfonat und 2-p-Toluidinyl-6-naphthalensulfonat. Andere Farbstoffe umfassen 3-Phenyl-7-isocyanatocoumarin, Acridine wie 9-Isothiocyanatoacridin-Orange, N-(p-(2-Benzoxyzolyl)-phenyl)-maleimid, Benzoxydiazole, Stilbene, Pyrene. Bevorzugte Fluorophore sind weiterhin SYBR Green, Hex, TET, VIC, JOE, NED, Redmond Red, Alexa Red, Cascade Blue, Yakima Yellow, Cy3, Cy3.5, Tamra/Cy3, Texas Red, ROX, Cy5, Cy5.5, Carboxyrhodamine, LC705 und/oder LC640. Als Quencher können beispielsweise weiterhin eingesetzt werden Tamra, Rhodamin, BHQ1 bis BHQ3, Dansyl, Dabcyl, ElleQuencher und/oder Methylorange. Bevorzugt kann auch eine Konjugation der Nukleinsäureproben mit Minor Grove Binder (MGB) sein. Derartige Strukturen sind beispielsweise in Kutyavin et al., 2000, Nucleic Acids Research beschrieben und sind in den Offenbarungsgehalt der Erfindung mit aufgenommen.

Erfindungsgemäß kann man zwei Arten von Quencherprozessen oder Prozessen, die eine Fluoreszenzstrahlung modifizieren, unterscheiden, einmal die dynamische Fluoreszenzlöschung durch Kollisionsprozesse und die statische Fluoreszenzlöschung durch Komplexbildung zwischen dem Fluorophor, das heißt dem Marker oder der Sonde und den Quencher- oder Löscher-Molekülen des Sonden-Quenchers. Das Quenching führt demgemäß zu einer Erniedrigung der Quantenausbeute, die durch Fluoreszenzanregung der markierten Sonde detektiert werden kann. Es ist aber beispielsweise auch möglich, dass die Sonden bei einer sehr hohen Konzentration, beispielsweise auf einem bestimmten Nukleinsäureabschnitt, zum so genannten Selbstquenching neigen, das heißt, dass die einzelnen Moleküle in ihrer Bewegung so gestört werden, dass ebenfalls ein Quenchingeffekt - bedingt durch die hohe Sondendichte - auftritt.

Denn im Sinne der Erfindung kann z. B. ein separat generiertes Fluoreszenzsignal, ein Fluoreszenzresonanzenergietransfer (FRET)-Signal als auch ein "gequenchtes" Signal dazu dienen, eine AKAP-PKA zu detektieren, bevorzugt ist ein FRET-Signal. Das FRET-Signal wird insbesondere durch die Verwendung der Fluoreszenzstoffe CFP und YFP gewonnen.

Mit den beiden bereitgestellten Plasmiden wird eine Zelle transfiziert. Die Transfektion in Sinne der Erfindung kann über chemische, physikalische und/oder biologische Transfektionsmethoden vorgenommen werden. Die chemische Transfektion kann beispielsweise durch den Einsatz von DEAE-Dextran, durch Dendrimere oder durch die Verwendung von Calciumphosphat vorgenommen werden. Bei der physikalischen Transfektion ist es beispielsweise möglich, mit Hilfe der Elektroporation die Membranen der Zellen so zu modifizieren, dass sie die zu transfizierende Plasmid-DNA aufnehmen. Eine weitere Methode der physikalischen Transfektion ist beispielsweise die Mikroinjektion oder die Einschleusung von DNA durch Beschuss mit beispielsweise Goldpartikeln. Methoden der biologischen Transfektion sind beispielsweise die rezeptorvermittelte Transfektion, die durch virale Komponenten unterstützte rezeptorvermittelte Transfektion und die Lipofektion. Dem Fachmann sind verschiedene Methoden zur Transfektion bekannt. Die Zellen, an denen die Transfektion vorgenommen werden kann, können prokaryotische oder eukaryotische Zellen sein, beispielsweise Bakterien-, Hefe-, Insekten-, Pflanzen- oder Säugerzellen oder aber auch Organismen wie transgene Tiere oder Pflanzen. In den eukaryotischen Systemen sind die Säugerzelllinien NSO, SP2/0, CHO-K1, CHO dhfr-, COS-1, COS-7, K562, Percy 6 oder bevorzugt HEK293-Zellen bevorzugt CD8-Zellen, LCCPK1, HeLazellen, MDCK2-Zellen, MCF7, Fibroblasten, MCF7, NIH3T3.

Nachdem die Zellen mit beiden Plasmiden unter den dem Fachmann bekannten Bedingungen kotransfiziert wurden, werden die beiden Fusionsproteine aus AKAP und erstem fluoreszierenden Protein und aus der regulatorischen Untereinheit einer Proteinkinase und dem zweiten fluoreszierenden Protein exprimiert. Sofern die exprimierten Fusionsproteine interagieren, kann dies auf Grund der Wechselwirkung der fluoreszierenden Proteine mit der Fluoreszenzresonanzenergietransfer-Technik, insbesondere in lebenden Organismen, wie in Zellen detektiert werden. Die Fluoreszenzresonanzenergietransfer-Technik beruht auf einem Energietransfer des ersten fluoreszierenden Proteins zu dem zweiten fluoreszierenden Protein, der jedoch nur dann zustande kommt, wenn sich beide Fusionsproteine in unmittelbarer Nähe zueinander befinden. Die Fusionsproteine erreichen diese Nähe insbesondere dann, wenn das AKAP-Protein direkt an die regulatorische Untereinheit der Proteinkinase bindet. In diesem Fall kann ein Fluoreszenzresonanzenergietransfer nachgewiesen werden.

Das System kann auch für die Identifikation von Substanzen genutzt werden, die die Interaktion zwischen AKAP und regulatorischen Untereinheiten, bevorzugt RIIα, aber auch RIIß, RIα und β der PKA, inhibieren.

Durch die Bereitstellung des erfindungsgemäßen Verfahrens kann der Fachmann das Verfahren beliebig modifizieren. Insbesondere ist es möglich, zu überprüfen, ob bestimmte Moleküle die Wechselwirkung von AKAP und PKA, insbesondere AKAP18δ und RIIα, beeinflussen. Hierzu kann das erfindungsgemäße Verfahren beispielsweise einmal in Gegenwart und einmal ohne das zu untersuchende Molekül durchgeführt werden, wobei der Vergleich des mit dem und ohne das zu untersuchende Molekül durchgeführten Verfahrens einen Hinweis auf den Inhibitionscharakter des Moleküls gibt. Sofern z. B. kein FRET-Signal in Gegenwart des Moleküls gemessen wird, ist dies ein Hinweis darauf, dass das Molekül die Wechselwirkung zwischen AKAP und PKA inhibiert.

Dem Fachmann ist bekannt, durch welche Kontrollversuche er ausschließen kann, dass das Molekül den FRET selbst unterdrückt; weiterhin ist ihm bekannt, wie er detektieren kann, ob das Molekül AKAP, PKA-Untereinheiten oder deren spezielle Wechselwirkung beeinflusst, vorzugsweise inhibiert.

Die Identifizierung spezifischer AKAP-Inhibitoren hat ein großes therapeutisches Potential. AKAP-PKA-Interaktionen spielen bei verschiedenen exozytotischen Prozessen eine Rolle, deren Fehlregulation zur Entstehung von Krankheiten wie Diabetes insipidus, Diabetes mellitus, Bluthochdruck, Magenulzera oder Schilddrüsenerkrankungen führt. Bei der Herzinsuffizienz kommt es zu einer PKA-vermittelten Hyperphosphorylierung eines Ionenkanals, des Ryanodinrezeptors (Calciumkanal). Substanzen, die bestimmte AKAP spezifisch inhibieren, könnten als Pharmaka bei diesen Erkrankungen eingesetzt werden.

Das Verfahren ist außerdem geeignet, die Membranpermeabilität von Peptiden zu erfassen. Bislang gibt es keine Möglichkeit einen Membrantransfer von Peptiden direkt nachzuweisen bzw. zu quantifizieren. Dies kann insbesondere erreicht werden, indem ein Konjugat aus dem zu untersuchenden Molekül, insbesondere ein Peptid und S-Ht31 bzw. ein Gemisch mit dem Peptid Ht31 hergestellt wird. Es ist jedoch jede RII-Bindungsdomäne jedes AKAPs möglich.

In einer besonderen Ausführungsform der Erfindung wird die Zelle mit einem membranpermeablem Peptid in Kontakt gebracht. Hierbei ist es beispielsweise möglich, dass die Fluoreszenzresonanzenergietransfer-Messung einmal ohne Zugabe des membranpermeablen Peptids und einmal mit Zugabe des membranpermeablen Peptids durchgeführt wird, wodurch detektiert werden kann, ob das membranpermeable Peptid die AKAP-PKA-Interaktion modifiziert, insbesondere unterbindet. Eine kontinuierliche Abnahme des Fluoreszenzresonanzenergietransfer-Signals während der Messung bedeutet beispielsweise eine Inhibition der Interaktion zwischen AKAP und der regulatorischen Untereinheit der Proteinkinase in Gegenwart des membranpermeablen Peptids. Es ist weiterhin auch möglich, bekannte membranpermeable Peptide einzusetzen, die die Interaktion zwischen AKAP und PKA inhibieren, wobei bei den membranpermeablen Peptiden bestimmte Modifikationen, wie beispielsweise Aminosäuredeletionen oder Substitutionen, untersucht werden, um einen Hinweis zu erhalten, welche Aminosäuren, in einem membranpermeablen Peptid essentiell sind, um die AKAP-PKA-Interaktion zu unterdrücken bzw. zu fördern.

Das Neue des erfindungsgemäßen Verfahrens besteht in der Visualisierung der AKAP-PKA-Interaktion in einer lebenden Zelle und in der Möglichkeit, den AKAP-PKA-Komplex einem zellulären Kompartiment zuzuordnen.

Das Verfahren ist jedoch sehr viel breiter verwendbar. Es ermöglicht ein Hochdurchsatzverfahren zur Identifikation und quantitativen Analyse von Substanzen, die die AKAP-PKA-Interaktion beeinflussen. Darüber hinaus kann die Membrangängigkeit von Peptiden bestimmt werden.

Die Erfindung betrifft auch die Verwendung der erfindungsgemäßen Nukleinsäure, des Vektors, der Wirtszelle, des Polypeptids, des Erkennungsmoleküls, der pharmazeutischen Zusammensetzung, des Kits und/oder des erfindungsgemäßen Verfahrens zur Detektion einer AKAP-PKA-Interaktion, einer AKAP- und/oder PKA-Inhibition und/oder eines membranpermeablen Peptids. Durch die Bereitstellung der genannten erfindungsgemäßen Strukturen und Verfahren hat der Fachmann die Möglichkeit, diese in zahlreichen Bereichen der Grundlagenforschung und Klinik einzusetzen. Es kann beispielsweise geprüft werden, ob ein Molekül ein AKAP- oder PKA-Inhibitor ist. Weiterhin kann geprüft werden, ob ein Molekül die Wechselwirkung von AKAP und PKA modifiziert. Weiterhin kann detektiert werden, ob ein Molekül, insbesondere ein Peptid, membranpermeabel ist.

Im Folgenden soll die Erfindung anhand von Beispielen näher erläutert werden, ohne auf diese Beispiele beschränkt zu sein.

### Beispiele

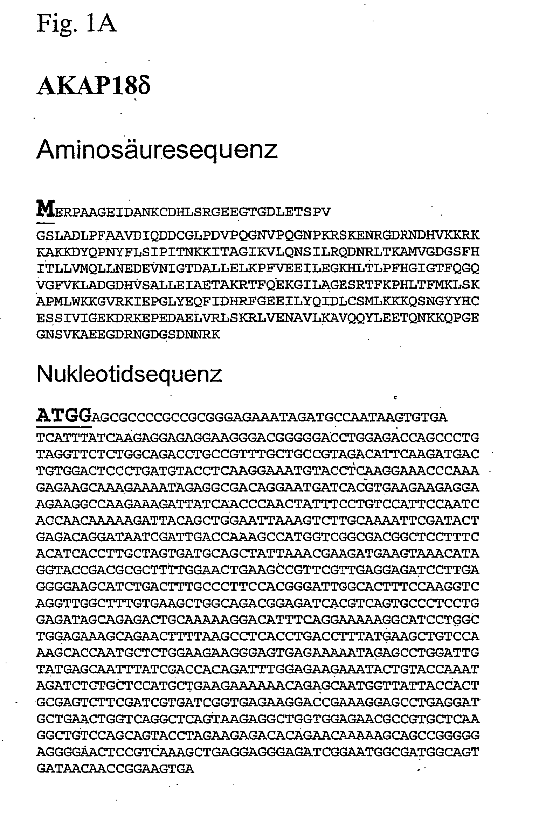

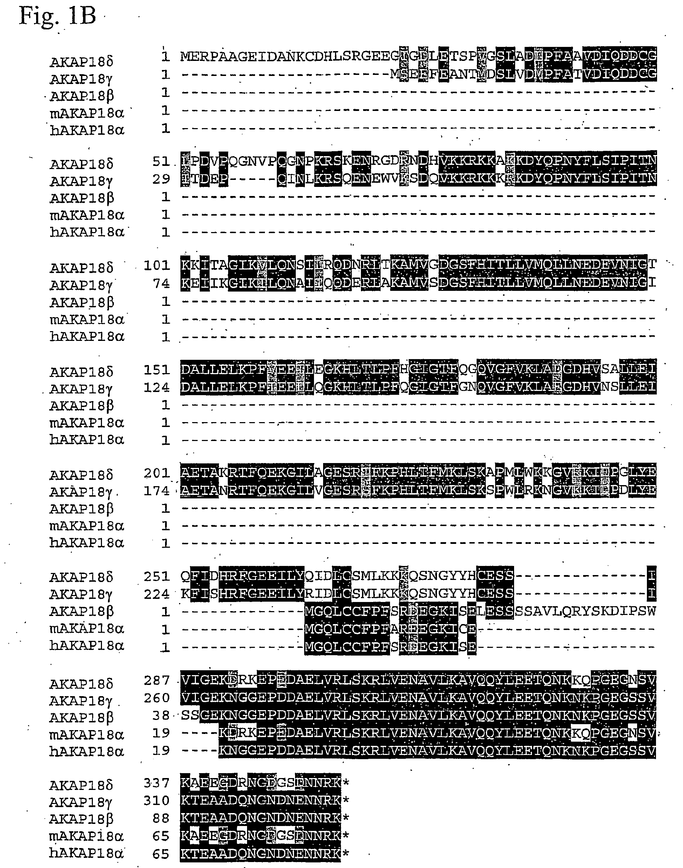

Es wurde zunächst die cDNA einer neuen Spleißvariante des Proteinkinase A-Ankerproteins (AKAP) AKAP18 identifiziert und isoliert (Fig. 1). Diese Variante wird als AKAP18δ bezeichnet. Die AKAP18δ-cDNA wurde in den kommerziell erhältlichen Vektor pECFP (BD Biosciences, Clontech Heidelberg) kloniert. Die cDNA der regulatorischen Untereinheit RIIα der humanen Proteinkinase A, die von Prof. Dr. K. Tasken (Universität Oslo) zur Verfügung gestellt wurde, wurde in den kommerziell erhältlichen Vektor pEYFP (BD Biosciences, (Clontech, Heidelberg) kloniert. Eukaryotische HEK293-Zellen (GBF, Braunschweig) wurden mit den Plasmiden kotransfiziert.

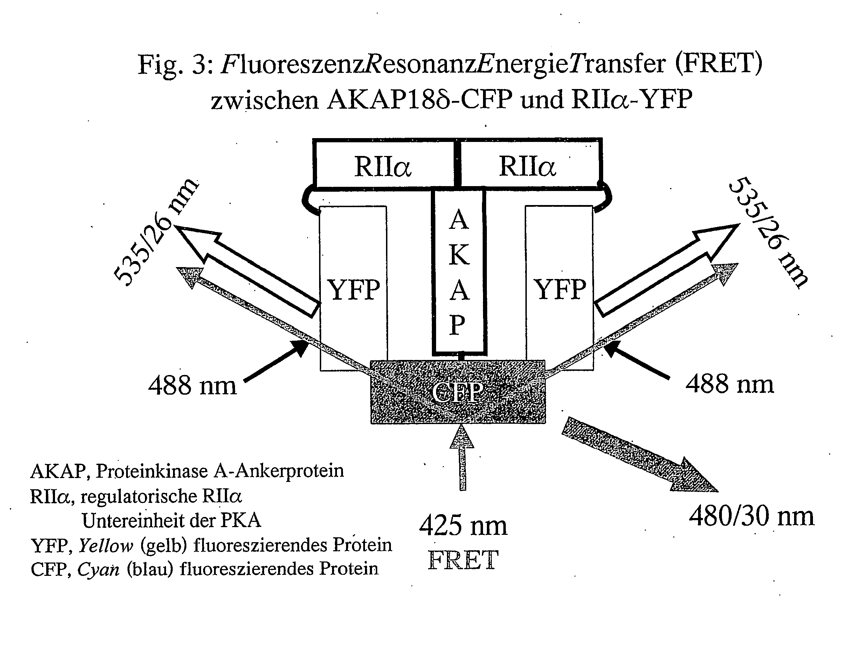

Die Interaktion der exprimierten Fusionsproteine AKAP18δ-CFP und RIIα-YFP wurde mittels der FluoreszenzResonanzEnergieTransfer (FRET)-Technik in den HEK293-Zellen gemessen (schematische Darstellung in Fig. 3). Die FRET-Signale beruhen auf einem Energietransfer von CFP zu YFP, der nur zustande kommt, wenn sich beide Proteine in unmittelbarer Nähe zueinander befinden (< 10 nM). CFP und YFP erreichen diese Nähe nur, wenn AKAP18δ die RIIα-Untereinheit direkt bindet. In diesem Fall kann ein FRET nachgewiesen werden. Die Spezifität der Interaktion von AKAPs18δ und RIIα kann dadurch überprüft werden, dass FRET in Gegenwart des membranpermeablen Peptids S-Ht31, das die Interaktion zwischen dem AKAP und der RII-Untereinheit verhindert, gemessen wird. Eine kontinuierliche Abnahme des FRET-Signals während der Messung bedeutet eine Inhibition der Interaktion zwischen AKAP185 und RIIα.

Die Sequenz des Peptids S-Ht31 entspricht der der PKA-Bindungsdomäne (RII-Bindungsdomäne) des AKAP Ht31. Es bildet eine amphipathische Helix aus und bindet kompetitiv an die regulatorischen PKA-Untereinheiten. Dadurch verhindert es die Interaktion mit AKAP18δ. Die Membranpermeabilität erhält das Peptid durch die Kopplung an einen Stearatrest am N-Terminus (Klussmann et al. J. Biol. Chem. 274, 4934-4938, 1999). Peptide mit der gleichen Aminosäuresequenz (Ht31), die keinen Stearatrest tragen und damit nicht membranpermeabel sind, verändern das FRET-Signal in dem System nicht. Ebenso verändert Stearat-gekoppeltes S-Ht31-Peptid, das durch Einfügen von zwei Prolinen, die die amphipathische Helix stören, das FRET-Signal nicht.

Teil der Charakterisierung eines neu identifizierten AKAP ist der Nachweis, dass es in vivo als AKAP, das heißt als PKA-Interaktionspartner, fungiert. Das erfindungsgemäße Verfahren erlaubt es, die Interaktion von AKAP18δ und den RIIα-Untereinheiten der PKA in lebenden Zellen nachzuweisen. Es ist damit dazu geeignet, den Nachweis zu erbringen, dass AKAP18δ als AKAP in vivo fungiert. Gleichzeitig lässt das System Rückschlüsse auf die intrazelluläre Lokalisation des AKAPI8δ-RIIα-Komplexes zu.

AKAP sind eine Familie von über 50 Proteinen, deren Funktion bislang nicht durch spezifisch interagierende Substanzen manipuliert werden kann. Das Peptid S-Ht31, mit dem die Bindung von AKAP18δ und RIIα inhibiert wurde (s.o.), ist bisher die einzige bekannte Substanz, die eine AKAP-Funktion beeinflusst. Es inhibiert die AKAP-PKA-Interaktion, allerdings entkoppelt es die Bindung zwischen jeglichem AKAP und allen regulatorischen PKA-Untereinheiten. Mit diesem entwickelten System sollen membranpermeable Peptide sowie niedermolekulare, nichtpeptidische Substanzen in gezielten Hochdurchsatzuntersuchungen mittels FRET-Messungen identifiziert werden, die die Interaktion zwischen AKAPs18δ und RIIα spezifisch inhibieren.

### Herstellung von Plasmiden, die für die Fusionsproteine AKAP18δ-CFP und RIIα-YFP kodieren

Die kodierende Region der von uns identifizierten AKAP18δ-cDNA (Fig. 1; Sequenz AKAPs18δ) wurde mittels der Polymerasekettenreaktion (PCR) amplifiziert. Dazu wurden *forward*-Primer (Position in AKAP,18δ: bp 57-76) mit der Sequenz 5' *CTC GAG CTC AAG CTT CGA ATT CTG ATG GAG CGC CCC GCC GCG GG* 3' und *reverse*-Primer (Position in AKAP,18δ: bp 1095 - 1118) mit der Sequenz 5' *GGC GAC CGG TGG ATC CCG GGC CCG GTT GTT ATC ACT GCC ATC GCC* 3', die eine EcoRI- bzw. eine BamHI-Restriktionsschnittstelle tragen, eingesetzt. Als Polymerase wurde der *Advantage cDNA polymerase*-Mix nach Herstellerangaben eingesetzt. Der benötigte 10x PCR-Puffer wurde mit dem *Advantage cDNA polymerase*-Mix mitgeliefert. Die Nukleotide dATP, dCTP, dGTP und dTTP wurden als dNTP-Mix in den PCR-Ansatz pipettiert (Reaktionsansatz siehe unten).

Die für RIIα kodierende cDNA wurde mittels PCR aus dem Plasmid amplifiziert. Dazu wurden *forward*-Primer (Position in RIIα : bp 190 - 210) mit der Sequenz 5' *TCA GAT CTC GAG CTC AAG CTT CGA ATT CTG ATG AGC CAC ATC CAG ATC CCG* 3' und *reverse*-Primer (Position in RIIa: bp 1382 - 1401) mit der Sequenz 5' *GAC CGG TGG ATC CCG GGC CTG CCC GAG GTT GCC CAG AT* 3', die eine XhoI- bzw. eine BamHI-Restriktionsschnittstelle tragen, eingesetzt. Als Polymerase wurde wieder der *Advantage cDNA polymerase*-Mix eingesetzt. Ebenso wurden der oben beschriebene 10x PCR-Puffer und der dNTP-Mix eingesetzt.

Die PCR-Reaktionen zur Amplifikation von AKAP185 und RIIα wurden wie folgt angesetzt:

| | |
|---|---|
| DNA | 5 µl |
| dNTP-Mix [10 µM] | 1 µl |
| Forward-Primer [10 µM] | 1 µl |
| Reverse-Primer [10 µM] | 1 µl |
| *Advantage cDNA polymerase*-Mix (5 Einheiten/µl) | 0,2 µl |
| H₂O | 41,8 µl |
| Gesamtvolumen | 50 µl |

### Reaktionsbedingungen:

| | |
|---|---|
| 1 Zyklus | 95°C, 5 min |
| 30 Zyklen | 94 °C, 30 sec |
| | 58°C, 30 sec |
| | 72 °C, 2 min |
| 1 Zyklus | 72°C 10 min |
| 4°C , ∞ | |

Das erhaltene AKAP18δ-cDNA-Amplifikat (Länge: 1061 bp) wurde mit den Restriktionsenzymen EcoRI und BamHI behandelt, das erhaltene RIIα-cDNA-Amplifikat (Länge: 1211 bp) mit den Restriktionsenzymen XhoI und BamHI. Anschließend wurden die Ansätze in einem Agarosegel aufgetrennt und die AKAP18δ- bzw. RIIα-Amplifikate mittels der Geneclean-Methode aus dem Gel eluiert.

Die AKAP18δ-cDNA wurde in das mit den Restrikzionsenzymen EcoRI und BamHI geschnittene Plasmid, das für das Cyan Fluoreszierende Protein (CFP) kodiert (pECFP, BD Biosciences), einkloniert. Die RIIα-cDNA wurde in das mit den Restriktionsenzymen EcoRI und BamHI geschnittene Plasmid, das für das Yellow Fluoreszierende Protein (YFP) kodiert (pEYFP, BD Biosciences), einkloniert. Die erhaltenen Plasmide kodieren somit für die Fusionsproteine AKAP18δ-CFP bzw. RIIα-YFP. Escherichia coli-Bakterien (Stamm JM109) wurden mit der Plasmid-DNA transformiert. Die in den Bakterien vermehrte Plasmid-DNA wurde mittel der Qiagen-Midi-Plasmidpräparationsmethode entsprechend den Herstellerangaben (Qiagen, Hilden) isoliert und mittels Transfektion in HEK293-Zellen eingeführt (s.u.).

HEK293-Zellen (GBF, Braunschweig) wurden auf Polylysinbeschichteten 30 mm-Deckgläschen in Dulbecco's Minimal Eagle Medium (DMEM) mit 10 % fötalem Kälberserum (FCS) kultiviert, bis eine Konfluenz von 40 - 60 % erreicht war. Die Zellen wurden mittels der Liptofectamine-Methode (Gibco Invitrogen, Karlsruhe) mit den AKAP18δ-CFP- und RIIα-YFP-Plasmiden (1 - 2 µg je DNA) transient transfiziert (Verhältnis von AKAP18δ-CFP-RIIα-YFP-Plasmid-DNA von 1:4).

### Fluoreszenzresonanzenergietransfer (FRET) -Messungen

HEK293-Zellen wurden transient mit den für RIIα-YFP und AKAPI8α-CFP kodierenden Plasmiden kotransfiziert. FRET-Messungen wurden 24 - 48 h nach der Transfektion an einem Epifluoreszenzmikroskop (Axiovert 200M, Carl Zeiss, Jena, Deutschland) durchgeführt. Die Daten wurden mittels der Openlab 2.25 Software (Improvision, Coventry GB) gespeichert. Die Fluoreszenz wurde bei einer Wellenlänge von 425 für CFP und 488 nm für YFP angeregte Die emittierte Fluoreszenz wurde bei Wellenlängen von 480/30 für CFP und 535/26 nm für YFP gemessen. FRET von CFP zu YFP wurde durch Anregung von CFP bei einer Wellenlänge von 425 nm und der Messung der Emission von YFP bei einer Wellenlänge von 535/26 nm bestimmt. Die unspezifische Hintergrundfluoreszenz wurde in einer Region ohne Zellen bestimmt und subtrahiert. Da die Einstellungen am Mikroskop unverändert blieben, konnte in allen Experimenten ein Verhältnis von 535/480 > 0,6 als positives FRET-Signal bezeichnet werden.

Bei FRET-Experimenten müssen falsch-positive Signale ausgeschlossen werden. Diese sind darauf zurückzuführen, dass bei der Anregung von CFP auch YFP angeregt wird. Des Weiteren strahlt die CFP-Emission auch in den Bereich der YFP-Emission hinein. Das Kontrollexperiment zum Nachweis richtig-positiver FRET-Signale erfolgt durch ein Akzeptor-Ausbleichungs-Protokoll (donor recovery after acceptor bleaching). Das Experiment wurde an einem inversen Epifluoreszenzmikroskop (Axiovert 100, Carl Zeiss, Jena, Deutschland) durchgeführt. Hierbei wird bei einer Anregung von 425 nm die Emission bei 480/30 und 535/26 nm mit einer 12 bit CCD-Kamera (Imago, TILL-Photonics, Martinsried, Deutschland) erfasst. Nach Bestimmung der Basis-Signale erfolgt dann eine starke Anregung von YFP bei einer Wellenlänge von 488 nm, die zum Verlust des YFP-Emissionssignals führt (acceptor bleaching). Die Emission von CFP, die bei einer Wellenlänge von 480/30 nm bestimmt wird, steigt sofort an, da FRET unterbrochen wird (donor recovery). In diesem System musste YFP durch mehrmalige starke Anregung bei einer Wellenlänge von 488 nm ausgeblichen werden, da die Lichtquelle nicht energiereich genug ist. Das Ausbleichen und damit der Verlust des YFP-Emissionssignals geschieht somit stufenweise. Dadurch steigt die Emission von CFP, die bei einer Wellenlänge von 480/30 nm bestimmt wird, kontinuierlich und nicht in einem einzigen Schritt an. Der Anstieg der CFP-Emission wird daher über einen Zeitraum von etwa 120 sec nach Beginn der Akzeptorausbleichung gemessen.

Um die Spezifität des FRET weiter zu untersuchen und zu testen, ob sich die kotransfizierten HEK293-Zellen dazu eignen, membranpermeable Substanzen zu identifizieren, die die Interaktion zwischen RIIα-YFP und AKAP18δ-CFP modulieren, wurden die Zellen mit dem membranpermeablen Peptid S-Ht31(100 µM) inkubiert, das generell AKAP-RII-Interaktionen hemmt. Zur Kontrolle wurden die Zellen mit dem Peptid S-Ht31-P, das keinen Einfluss auf die AKAP-RII-Interaktion hat (Klußmann et al., J. Biol. Chem. 274, 4934-4938, 1999), inkubiert. FRET-Messungen wurden in Intervallen von 10 min über insgesamt 90 min durchgeführt.

### Ergebnis

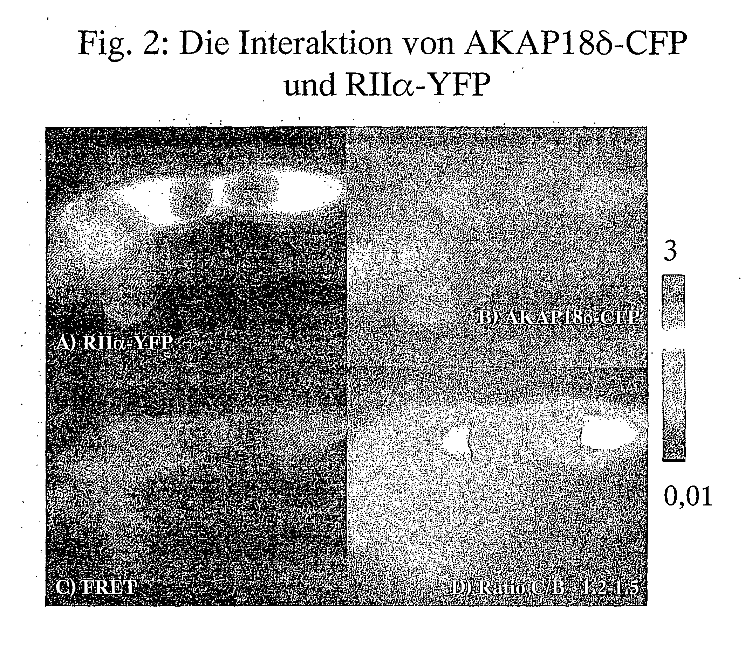

Zum direkten Nachweis einer Interaktion von AKAP18δ und den regulatorischen RIIα-Untereinheiten der PKA in vivo wurden HEK293 mit Plasmiden kotransfiziert, die für AKAP18δ-CFP und RIIα-YFP kodieren. Fig. 2 belegt die Koexpression beider Proteine in den gleichen Zellen. Die Expression von RIIα-YFP wurde durch Anregung bei 488 nm und Messung der emittierten Fluoreszenz bei 535/26 nm nachgewiesen (Fig. 2A), die von AKAP18δ-CFP durch Anregung der Fluoreszenz bei 425 nm und Messung der emittierten Fluoreszenz bei 480/30 nm (Fig. 2B). Beide Fusionsproteine zeigten eine diffuse, zytosolische Verteilung. Anschließend wurden FRET-Messungen an den gleichen Zellen durchgeführt. Dazu wird der Donor CFP bei einer Wellenlänge von 425 nm angeregt. Hält sich ein geeigneter Akzeptor in seiner unmittelbaren Nähe auf (Abstand < 10 nm), führt die Anregung des Donors zu einem teilweisen Energietransfer auf den Akzeptor YFP, der dann bei einer Wellenlänge von 545 nm fluoresziert (schematische Darstellung in Fig. 3). Demnach wurden zum Nachweis von FRET die HEK293-Zellen (CFP) bei einer Wellenlänge von 425 nm angeregt und es wurde die Emission von YFP bei einer Wellenlänge von 535/26 nm gemessen. Fig. 2C zeigt eine ähnliche Verteilung der dargestellten YFP-Emission wie in Fig. 2A. Somit hat ein Energietransfer von CFP nach YFP stattgefunden. Fig. 1D zeigt die farbkodierte Darstellung der berechneten Ratio 535/480 von etwa 1.2 - 1.5 der FRET-Signale in diesen Zellen.

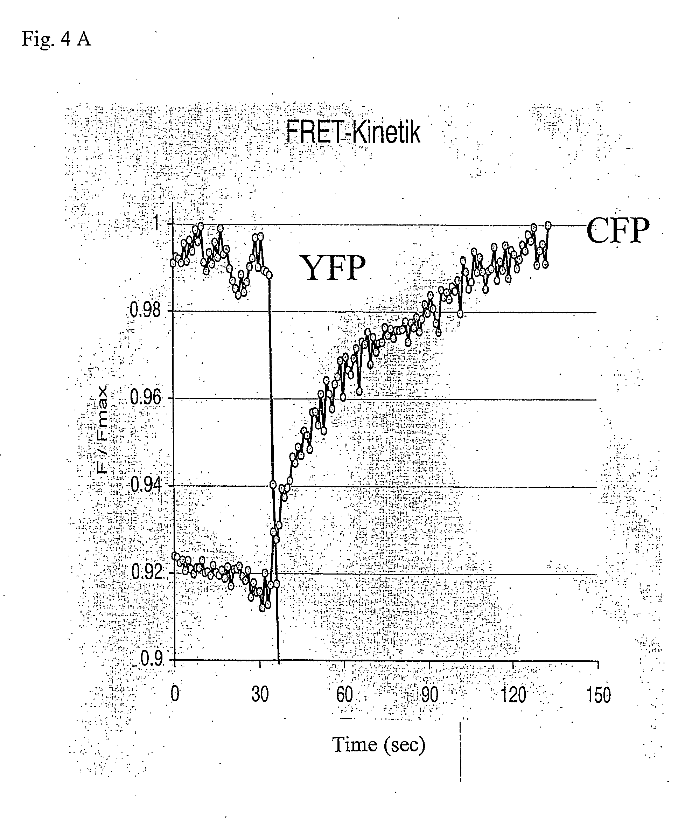

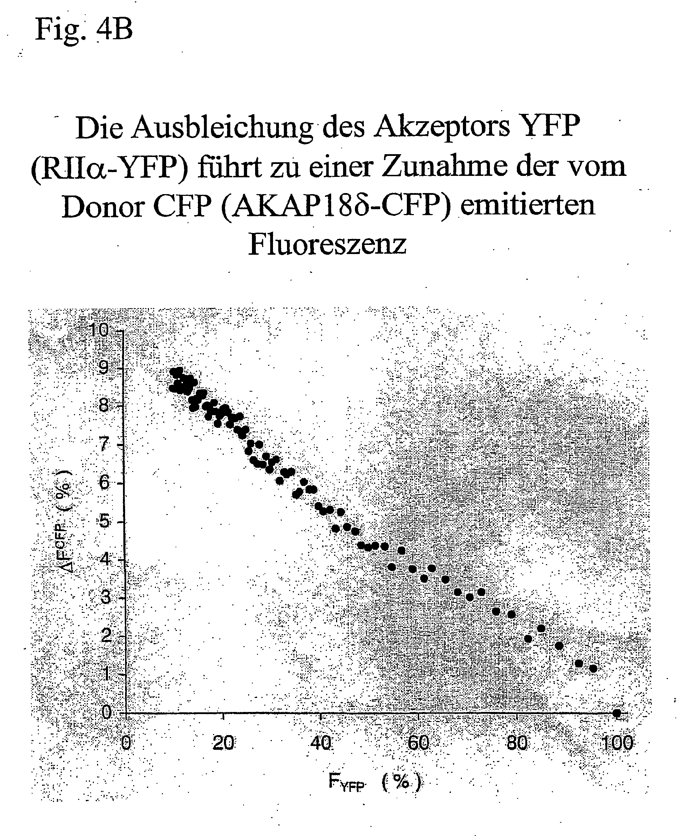

Die Spezifität der gemessenen FRET-Signale wurde mittels des Akzeptor-Ausbleichungs-Protokolls überprüft. Fig. 4A beschreibt die Kinetik der von YFP und CFP emittierten Fluoreszenz. Dargestellt ist das Verhältnis von F/Fₘₐₓ in Abhängigkeit von der Zeit (Zeit in sec). Fₘₐₓ entspricht der maximalen Emission von YFP bzw. CFP. Vor der Akzeptorausbleichung beträgt die F/Fmax von YFP annähernd 1, die von CFP etwa 0,92. Die Akzeptorausbleichung, beginnend nach 40 sec, führt zu einer starken Abnahme des Emissionssignals von YFP. Die F/Fₘₐₓ für CFP steigt von etwa 0.91 auf etwa 1 an. Aus dem Anstieg der CFP-Emission um etwa 10 % ergibt sich eine FRET-Effizienz von etwa 10 %. Die Regressionsanalyse (Fig. 4B) bestätigt diese Beobachtung. Diese Daten zeigen eine direkte Interaktion von AKAP18δ und RIIα.

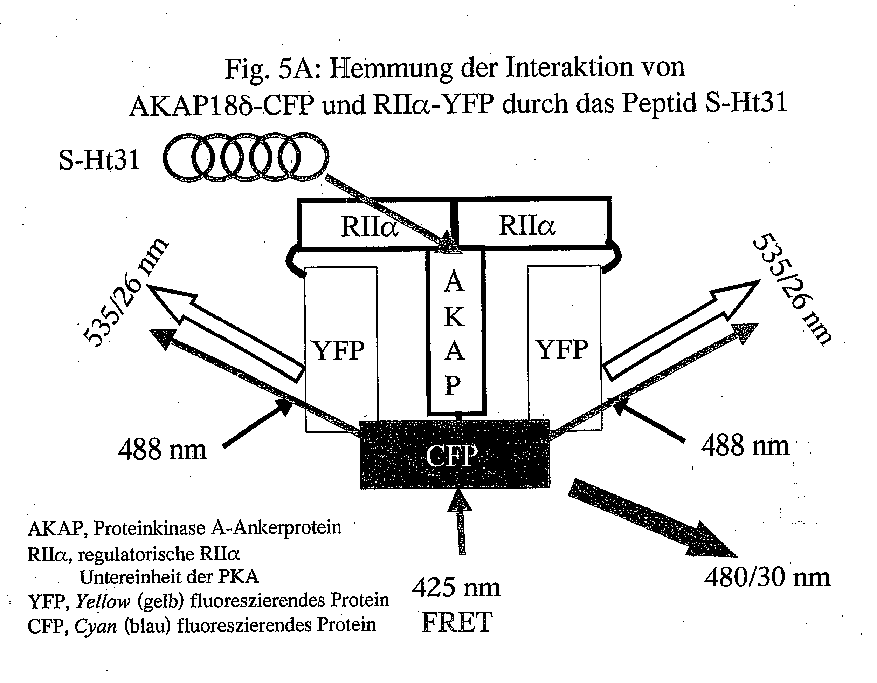

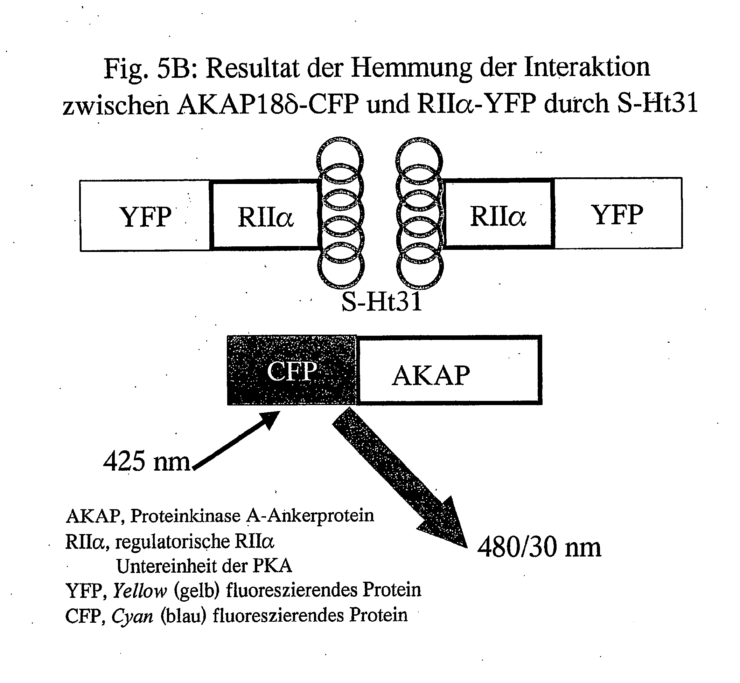

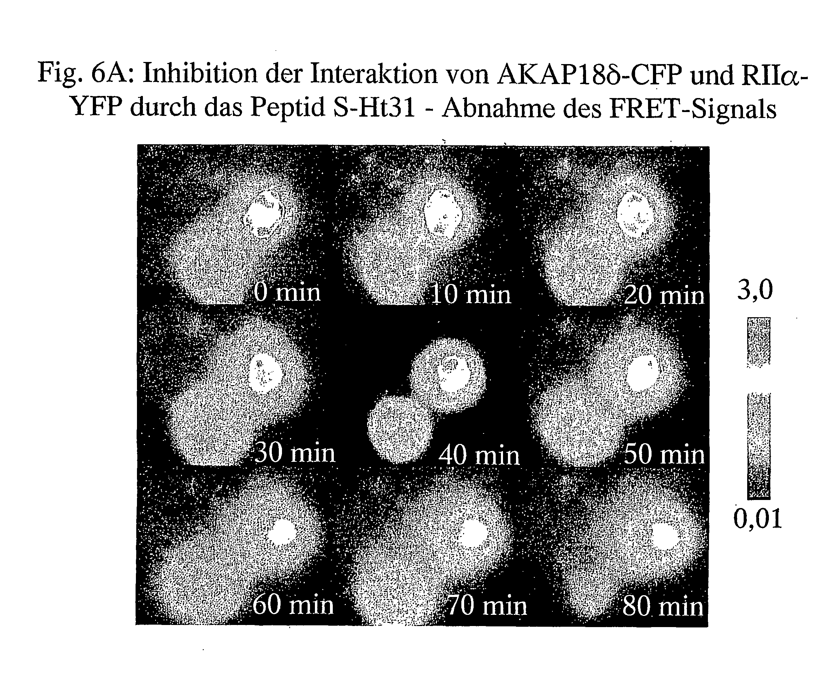

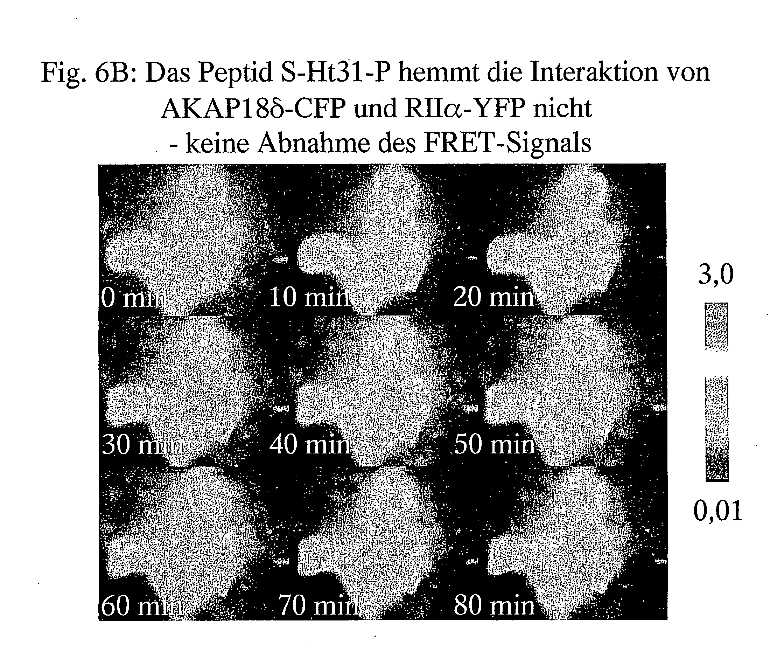

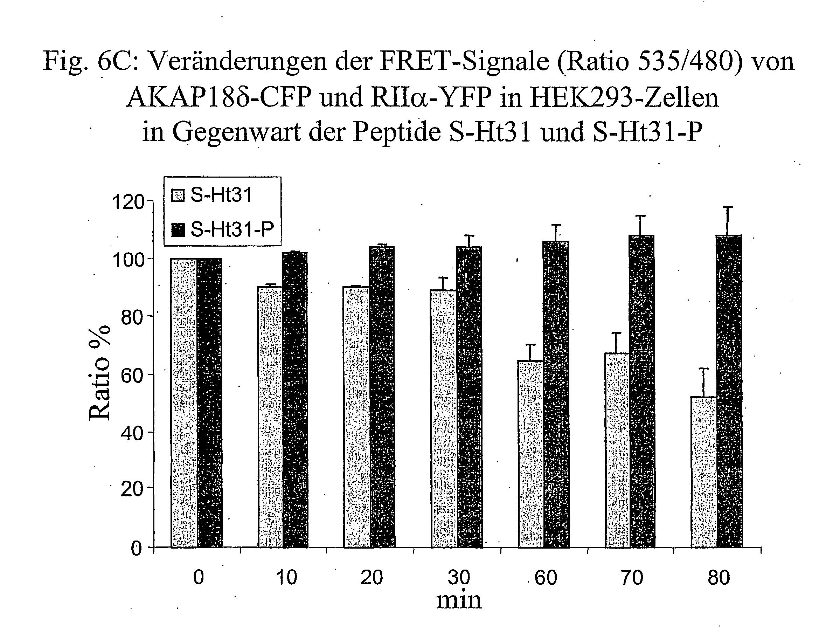

Um die Spezifität der Interaktion von AKAP18δ und RIIα und damit des FRET weiter zu untersuchen, aber auch um zu testen, ob sich die kotransfizierten HEK293-Zellen dazu eignen, membranpermeable Substanzen zu identifizieren, die die Interaktion von AKAP18δ und RIIα modulieren, wurden FRET-Messungen in Gegenwart des membranpermeablen Peptids S-Ht31 durchgeführt. Dieses Peptid hemmt generell die Interaktion zwischen AKAP und regulatorischen PKA-Untereinheiten (Klussmann et al., J. Biol. Chem. 274, 4934-4938, 1999; s. schematische Darstellung Fig. 5A und B). Fig. 6A zeigt das farbkodierte FRET-Signal (Verhältnis 535/480) von AKAP18δ zu RIIα in zwei HEK293-Zellen. Vor der Zugabe von S-Ht31 (Zeitpunkt 0 min) betrug die Ratio 535/480 etwa 1.3. Eine Abnahme des Verhältnisses 535/480 (weniger rot) korreliert direkt mit der Abnahme der Interaktion von CFP und YFP. Die Zugabe von S-Ht31 (100 µM, nach Zeitpunkt 0) induzierte eine Abnahme des Verhältnisses 535/480 nm um über 50 % innerhalb von 80 min. Der Mittelwert des Verhältnisses betrug etwa 0.35. Fig. 6B zeigt Zellen, die mit dem wirkungslosen Kontrollpeptid S-Ht31-P inkubiert wurden, das keinen Einfluss auf die AKAP-RII-Interaktion hat. Hier ändert sich das farbkodierte FRET-Signal kaum. Fig. 6C zeigt eine Zusammenfassung der erhaltenen Daten aus Fig. 6A und B. Dargestellt ist die Veränderung des FRET-Signals (in %) in Abhängigkeit von der Zeit. Die Graphik zeigt die an den Zellen beobachtete Veränderung des FRET-Signals in Gegenwart von S-Ht31 oder S-Ht31-P.

Diese Ergebnisse zeigen, dass HEK293-Zellen, die AKAP18δ-CFP und RIIα-YFP koexprimieren, ein geeignetes System zur Identifizierung membranpermeabler Substanzen darstellen, die die Interaktion zwischen diesem AKAP und RIIα inhibieren.

AKAP bindet regulatorische PKA-Untereinheiten über ein konserviertes Strukturmotiv (amphipathische Helix). Daher kann eine Interaktion jedes AKAP mit einer regulatorischen PKA-Untereinheit in diesem System mittels FRET bestimmt werden. Diese Möglichkeit bedeutet, dass das System für die Suche nach spezifischen, membranpermeablen Inhibitoren für jede AKAP-PKA-Interaktion nutzbar ist.

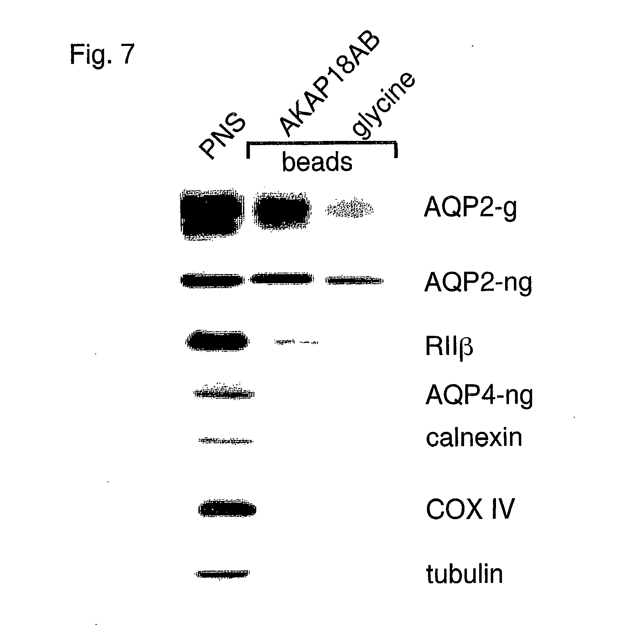

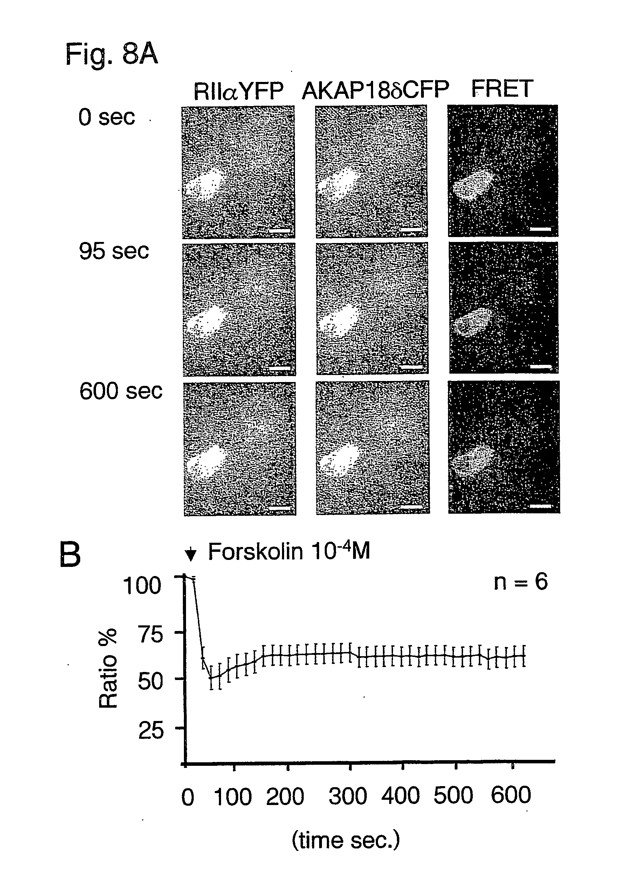

Die unterschiedliche Primärstruktur von AKAP18δ und AKAP18γ führt zu ihrer unterschiedlichen subzellulären und gewebespezifischen Verteilung und ist für ihre unterschiedlichen Funktionen verantwortlich. So kommt beispielsweise AKAP18γ im Kern von Maus-Oozyten vor und spielt möglicherweise bei der Gentranskription eine Rolle (Brown et al., 2003). Darüber hinaus wurde AKAP18γ in den zytosolischen und partikulären Fraktionen von nicht näher identifizierten Nierenzellen der Ratte gefunden. Die Identifizierung des/der Zelltyps/Zelltypen war bei den Experimenten nicht möglich, da Homogenate von gesamten Nieren verwendet wurden. Nach einer Ultrazentrifugation der Homogenate wurden Überstände und Sedimente-als zytosolische bzw. partikuläre Fraktionen bezeichnet; so beispielsweise von Trotter et al, (1999). Durch die Erfinder der anmeldungsgemäßen Lehre konnte gezeigt werden, dass AKAP18γ sowohl im Zytosol als auch im Kern von Hauptzellen der renalen Sammelrohre der Ratten vorkommt. In den Hauptzellen reguliert das antidiuretische Hormon Arginin-Vasopressin (AVP) die Wasserrückresorption durch Umverteilung des Wasserkanals Aquaporin-2 (AQP2) aus intrazellulären Vesikeln in die dem Urin zugewandte Seite der Plasmamembran (Klussmann et al., 2000). Die Funktion von AKAP18γ in den Hauptzellen ist unbekannt. AKAP18δ ist wie AKAP18γ im Zytosol und im Kern renaler Hauptzellen lokalisiert, aber im Gegensatz zu AKAP18γ kolokalisiert es mit AQP2 an intrazellulären Vesikeln (Fig. 7). Die Vesikel enthalten auch regulatorischen RIIß -PKA-Untereinheiten aber keine Proteine, die typischerweise bzw. ausschließlich an anderen Zellorganellen vorkommen (Fig. 7). AKAP18δ kann daher PKA an den Vesikeln verankert, eine Funktion die das bekannte AKAP18γ nicht erfüllen kann. Weiterhin konnte eine direkte Beteiligung des erfindungsgemäßen AKAP18δ an der Vasopressin-vermittelten Wasserrückresorption in den renalen Hauptzellen beispielsweise durch FRET-Experimente gezeigt werden. Als Modellsystem dienten hierbei CD8-Zellen (Valenti et al., 1996). Hierbei handelt es sich um eine permanente Zellinie aus Kaninchennieren, die stabil das AQP2 der Ratte exprimiert. Die Stimulation der Zellen mit Forskolin (direkter Aktivator der Adenylylzyklase, Überspringen der Vasopressinrezeptoraktivierung) führt analog der Stimulation nativer Hauptzellen durch Vasopressin zu einer Translokation von AQP2 in die apikale Plamamembran: Wie weiter unten ausgeführt, wurden die CD8-Zellen mit den erfindungsgemäßen AKAP188 CFP- und RIIα YFP-Konstrukten kotransfiziert. Die Stimulation der Zellen mit Forskolin induzierte nicht nur die Translokation von AQP2 in die apikale Plasmamembran sondern auch die Dissoziation des AKAPs und der PKA (Fig.8). Der molekulare Mechanismus der Dissoziation beruht möglicherweise auf einer Phosphorylierung einer PKA-Konsensusphosphorylierungssequenz in der PKA-Bindungsstelle des AKAPs. Diese Phosphorylierung verhindert dann aufgrund elektrostatischer Abstoßung die Bindung der PKA an AKAP18δ.

Neben diesen allgemeinen Unterschieden der Struktur und Funktion zwischen der bekannten und der neuen Spleißvariante konnten auch deutlich organspezifische Unterschiede gezeigt werden. Im Gegensatz zu der bekannten Spleißvariante verankert AKAP18δ die PKA an Ca ²⁺ -Kanälen und -Rezeptoren in Herzmuskelzellen

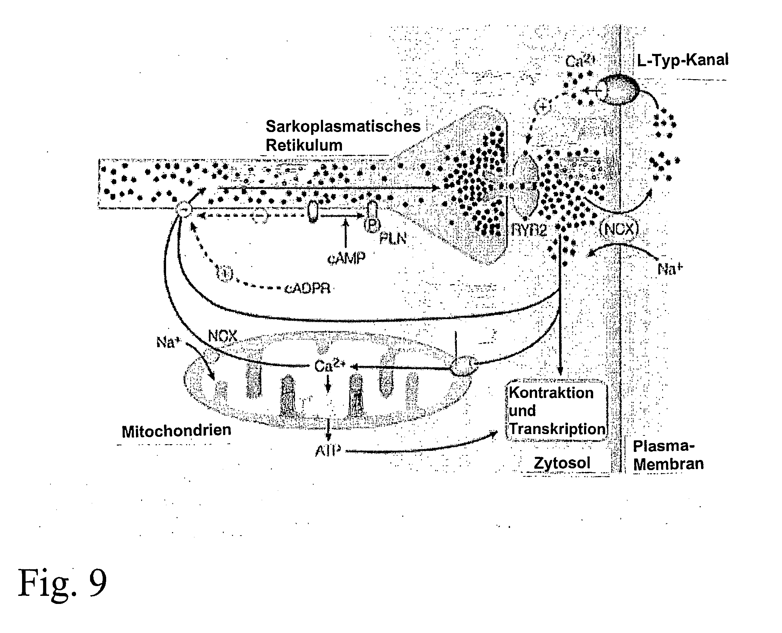

Die Herzmuskelkontraktion wird durch die Erhöhung des zytosolischen Ca²⁺ in Herzmuskelzellen hervorgerufen (Berridge et al., 2003; Fig. 9). Das Ca²⁺ gelangt durch L-Typ-Ca²⁺-Kanäle von außen in die Zellen nachdem ein Aktionspotential die Zellen erreicht hat und die Öffnung der in der Plamamembran lokalisierten Kanäle bewirkt hat. Das einströmende Ca²⁺ bindet an Ryanodin R2-Rezeptoren (RyR2) des sarkoplamatischen Retikulums (SR), aktiviert sie und induziert den Efflux von Ca²⁺ aus dem SR durch RyR2 in das Zytosol. Das zytosolische Ca²⁺ verursacht über mehrere Zwischenschritte die Kontraktion des Zytoskeletts und damit der Herzmuskelzelle. Die Ca²⁺-ATPase SERCA2a ist durch das Zurückpumpen des Ca²⁺ aus dem Zytosol in das SR wesentlich an der Beendigung der Kontraktion beteiligt. SERCA2a wird durch die Bindung des Proteins Phospholamban (PLN) inhibiert. Mit dem Anstieg des zytosolischen Ca²⁺ wird die Inhibition durch Dissoziation des PLN von SERCA2a aufgehoben.

Die Stärke der Herzmuskelzellkontraktion und damit die Stärke der Kontraktion des gesamten Herzmuskels wird durch die Menge des Ca²⁺ im Zytosol moduliert. Ein physiologischer Modulator der Ca²⁺-Menge in den Herzmuskelzellen ist z.B. Adrenalin, das an β-Rezeptoren auf der Oberfläche von Herzmuskelzellen bindet. Der Bindung an diese Rezeptoren folgt die Aktivierung der PKA, die L-Typ Ca²⁺-Kanäle, RyR2, PLN und das Zytoskelettprotein Troponin C phosphoryliert. Die Phosphorylierung von L-Typ Ca²⁺-Kanälen und RyR2 verstärkt ihre Ca²⁺-Leitfähigkeit und erhöht somit die zytosolische Ca²⁺-Konzentration. Phosphoyliertes Troponin C erhöht die Kontraktilität des Zytoskeletts. Die Phosphorylierung von PLB bewirkt seine Dissoziation von SERCA2a und dadurch einen verstärkten Rücktransport des Ca²⁺ aus dem Zytosol in das SR. Dadurch steht für die nächste Herzmuskelkontraktion vermehrt Ca²⁺ zum Einstrom in das Zytosol zur Verfügung. Die sog. β-Blocker, die z.B. zur Therapie des Bluthochdrucks eingesetzt werden, verhindern diese Phosphorylierungseignisse durch Blockade der β-Rezeptoren und senken somit die Herzkontraktilität und die Herzschlagfrequenz.

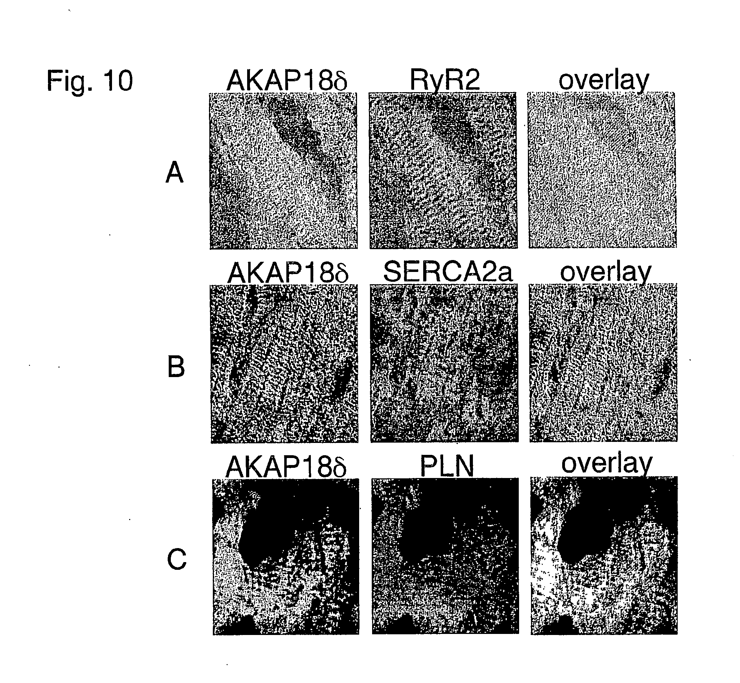

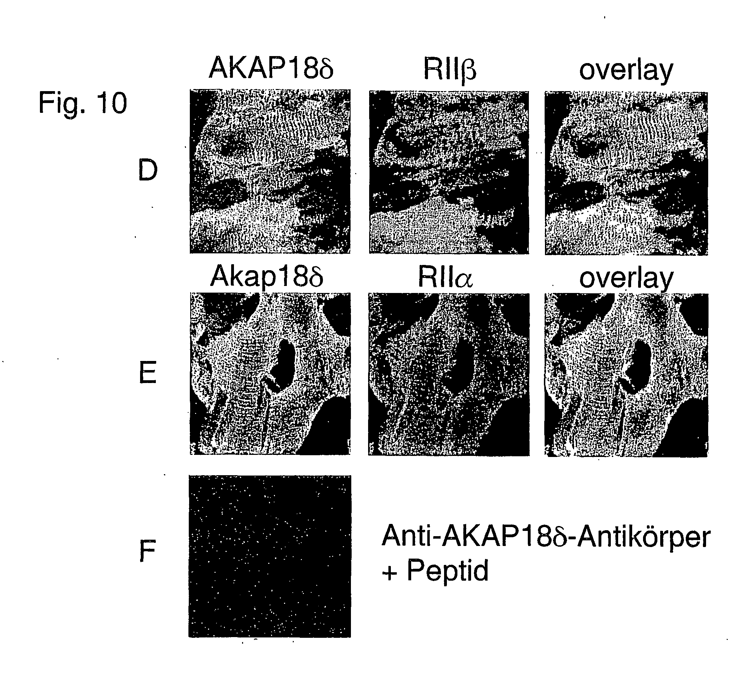

So konnte in erfindungsgemäßen Western Blot-Untersuchungen gezeigt werden, dass AKAP18δ im Herzen exprimiert wird, AKAP18γ hingegen nicht. So zeigen immunfluoreszenzmikroskopische Untersuchungen (Fig. 10) mit einem anti-AKAP18δ-Antikörper eine Kolokalisation von AKAP18δ mit RyR2, SERCA2a und PLN. Des Weiteren kolokalisiert AKAP18δ mit den regulatorischen RIIα- und β-Untereinheiten der PKA in den Herzzellen. Es konnte demgemäß gezeigt werden, dass AKAP18δ die PKA in räumlicher Nähe zu RyR2, SERCA2a und PLN verankert und deren Phosphorylierung ermöglicht.

AKAP18α, das auch als AKAP15 bezeichnet, ist eine weitere Spleißvariante des AKAP18-Gens. Das entsprechende Protein besteht aus 81 Aminosäuren. Es ermöglicht die Phosphorylierung von L-Typ Ca²⁺-Kanälen im Skelettmuskel von Kaninchen durch die Verankerung der PKA am L-Typ Ca²⁺-Kanal (Hulme et al., 2002).

Aus dem o. g. kann geschlussfolgert werden, dass AKAP18γ und AKAP18δ in ihrer Primärstruktur, in ihrer subzellulären Lokalisation, in der gewebsspezifischen Verteilung und ihrer Funktion deutliche Unterschiede aufweisen.

Fig. 7. AQP2, PKA und AKAP18δ und/oder ein 55 kDa AKAP kommen auf den gleichen intrazellulären Vesikeln vor. Die innere Medulla von Rattennieren wurde homogenisiert und die Nuklei und Zelldebris durch Zentrifugation entfernt. Der resultierende postnukleäre Überstand wurde mit affinitätsgereinigten anti-AKAP18δ Antikörpern inkubiert, die an eine Eupergit C1Z-Methacrylatmatrix gekoppelt (AKAP18AB beads). Nichtgesättigte Bindungsstellen wurden durch Inkubation mit Glycin blockiert. Zur Kontrolle wurden beads nur mit Glycin beschichtet (glycine beads). Unsere biochemischen Analysen zeigen, dass der anti-AKAP18δ-Antikörper in der partikulären Fraktion von Hauptzellen AKAP18δ und ein 55 kDa AKAP erkennt. Der Antikörper detektierte AKAP18γ in der löslichen Fraktion von Hauptzellen und in deren Kern. Glykosyliertes (g) und nichtglykosyliertes (ng) AQP2, regulatorische RIIß PKA-Untereinheiten und als Kontrolle Marker für die Plasmamembran (nichtglykosyliertes AQP4, AQP4-ng), das endoplasmatische Retikulum (calnexin), Mitochondrien (cytochrome c oxidase subunit IV, COX IV) und das Zytoskelett (tubulin) wurden mit kommerziell erhältlichen Antikörpern mittels Western blot detektiert.

Fig. 8. AKAP18δ spielt in der Signalkaskade, die zur Translokation von AQP2 in die apikale Plasmamembran renaler Hauptzellen führt, eine Rolle. A: CD8-Zellen wurden mit Plasmiden, die für RIIα-YFP und AKAP18δ-CFP kodieren kotransfiziert. FRET wurde vor und nach Stimulation der Zellen mit Forskolin (100 µM) gemessen. Hier dargestellt sind zwei repräsentative Zellen, die jeweils die zwei Fusionsproteine koexprimieren. FRET wurde vor der Forskolingabe (0 sec) und 95 und 600 sec später (95 sec and 600 sec) gemessen. Das FRET-Signal (Ratio 535/480 nm) ist in Falschfarben kodiert. B: Quantitative Analyse des Effekts von Forskolin auf das FRET-Signal (n = 6 cells). Masstab, 20 µm.

Fig. 9. Calciumsignale im Herzen (aus Berridge et al., 2003). Lokale Ca^{z+}-Signale sind für die Kontraktion und möglicherweise für die Gentranskription verantwortlich (Ca²⁺, rote Kreise). Ca²+-Signale (*Ca²⁺ signalling*) beginnen mit ihrer Amplifikation in der Nähe von L-Typ Ca²⁺-Kanälen an den T-Tubuli. Als T-Tubuli werden Regionen im Herzen bezeichnet, an denen sich die Plasmamembran der Herzzellen und die Membran des sarkoplasmatischen Retikulums (SR) in unmittelbarer Nähe befinden. Die Depolarisation der Plamamembran (T-tubule membrane) führt zu einer lokalen, pulsativen Erhöhung des zytosolischen Ca²⁺, das dann zum Ryanodin R2-Rezeptor 2 (RyR2) diffundiert und RyR2 aktiviert. Durch RyR2 gelangt dann Ca²⁺ aus dem SR in das Zytosol (cytoplasm) und diffundiert von dort bis es die Kontraktion versucht. Die Kontraktion wird beendet indem das Ca²⁺ durch den Na⁺/Ca²⁺-Austauscher (NCX) aus den Zellen herausgepumpt wird oder durch SERCA in das SR zurückgepumpt wird. Ein Teil des Ca²⁺ wandert über die Mitochondrien, wo es metabolische Vorgänge stimuliert, die ATP für die Kontraktion und die Transkription zur Verfügung stellen. Im SR gelangt das Ca²⁺ zielgerichtet zu der an den T-Tubuli lokalisierten SR-Membran (tunnelling) und wird für den nächsten Herzschlag wieder freigesetzt. Die Zirkulation des Ca²⁺ wird durch *second messenger* wie zyklisches AMP (cAMP) moduliert. Zyklisches AMP hebt die inhibitorische Wirkung von Phospholamban (PLN) auf. Ein anderer *second messenger,* zyklische ADP-Ribose (cADPR) aktiviert SERCA, so daß eine größere Menge freisetzbaren Ca²⁺ im SR vorliegt.

Fig. 10. Die Lokalisation von AKAP18δ, Ryanodin R2-Rezeptor 2 (RyR2), SERCA2a und Phospholamban (PLN), regulatorischen RIIα- und β-Untereinheiten der PKA in Herzzellen der Ratte. Es wurden 5 µm-Schnitte aus Rattenherzen hergestellt. AKAPs18δ wurde mit einem von uns herstellten und charakterisierten anti-AKAP18δ-Antikörper (Henn et al., Manuskript in Vorbereitung) und Cy3-gekoppelten Sekundärantikörpern detektiert. Die gleichzeitige Detektion der in der Abbildung genannten Proteine erfolgte mit kommerziell erhältlichen Antikörpern und Cy5-gekoppelten Sekundärantikörpern. Die Cy3- und Cy5-Fluoreszenzsignale (grün bzw. rot) wurde mit einem Laser scanning Mikroskop (LSM 510, Zeiss, Jena) aufgenommen. Die Kolokalisation von AKAP18δ mit den anderen Protein zeigt sich im Überlagerungsbild (overlay) anhand der Gelbfärbung.

### Literatur

Berridge,M.J., Bootman,M.D., and Roderick, H.L. (2003) Calcium signalling: Dynamics, homeostasis and remodelling. Nature Rev. Mol Cell. Biol., 4, 517-529.
Brown,R.L., August, S.L, Williams,C.J. and Moss,S.B. (2003) AKAP7γ is a nuclear RI-binding AKAP. Biochem. Biophys. Res. Comm., 306, 394-401.
Henn,V. Edemir, B., Stefan, E., Schmitt, R., Vossebein, L., Lorenz, D., Tamma, G., Beyermann, M., Krause, E., Herberg, F.W., Valenti, G., Bachmann, S., Rosenthal, W., and Klussmann, E. Evidence for a role of novel A-kinase anchoring protein 18 isoforms in the vasopressin-induced aquaporin-2 shuttle in renal principal cells. Manuskript in Vorbereitung.
Hulme,J.T., Ahn,M., Hauschka.S.D., Scheuer,T. and Catterall,W.A. (2002) A novel leücine zipper targets AKAP15 and cyclic AMP-dependent protein kinase to the C terminus of the skeletal muscle Ca2+ channel and modulates its function. J. Biol. Chem., 277, 4079-4087.
Klussmann,E., Maric,K. and Rosenthal,W. (2000) The mechanisms of aquaporin control in the renal collecting duct. Rev. Physiol. Biochem. Pharmacol., 141, 33-95.
Trotter,K.W., Fraser,I.D., Scott,G.K., Stutts,M.J., Scott,J.D. and Milgram,S.L. (1999) Alternative splicing regulates the subcellular localization of A-kinase anchoring protein 18 isoforms. J. Cell Biol., 147, 1481-1492.
Valenti,G., Frigeri,A., Ronco,P.M., D'Ettorre,C. and Svelto,M. (1996) Expression and functional analysis of water channels in a stably AQP2-transfected human collecting duct cell line. J. Biol. Chem., 271,24365-24370; correction (1997) J. Biol. Chem. 272, 26794.

## Patentansprüche

1. Isoliertes Nukleinsäuremolekül ausgewählt aus der Gruppe umfassend
a) ein Nukleinsäuremolekül umfassend eine Nukleotidsequenz ausgewählt aus der Gruppe bestehend aus der Nukleotid sequenz von Fig. 1A oder deren komplementäre Nukleotidsequenz,
b) ein Nukleinsäuremolekül, welches mit einer Nukleotidsequenz gemäß a) unter stringenten Bedingungen hybridisiert und für ein Protein kodiert, welches mit AQP2 an intrazellulären Vesikeln in renalen Hauptzellen lokalisierbar ist,
c) ein Nukleinsäuermolekül, das in Folge des genetischen Codes zu einer Nukleotidsequenz gemäß a) degeneriert ist.

2. Nukleinsäuremolekül nach Anspruch 1,
**dadurch gekennzeichnet, dass**
die unter b) angegebene Nukleotidsequenz mindestens 80%, bevorzugt 90% homolog zu einer unter a) angegebenen Nukleotidsequenz ist.

3. Nukleinsäuremolekül gemäß Anspruch 1 oder 2,
**dadurch gekennzeichnet, dass**
es eine genomische DNA, eine cDNA und/oder eine RNA ist.

4. Vektor umfassend ein Nukleinsäuremolekül gemäß einem der Ansprüche 1 bis 3.

5. Wirtszelle umfassend den Vektor gemäß Anspruch 4.

6. Polypeptid, kodiert durch ein Nukleinsäuremolekül gemäß einem der Ansprüche 1 bis 3.

7. Polypeptid mit der Aminosäuresequenz von Fig 11.

8. Antikörper, gerichtet gegen ein Polypeptide gemäß Anspruch 7 ,wobei der Antikörper AKAP18 delta spezifisch bindet.

9. Antikörper nach Anspruch 8 ,
**dadurch gekennzeichnet, dass**
der Antikörper ein Antikörperfragment, insbesondere Fab, Fab', F(ab')₂, Fᵥ oder Einzelkettenantikörper und/oder ein oligomerer, ein reduzierter, ein oxidierter und/oder ein markierter Antikörper ist.

10. Pharmazeutische Zusammensetzung,
**dadurch gekennzeichnet, dass**
sie ein Nukleinsäuremolekül gemäß einem der Ansprüche 1 bis 3, einen Vektor gemäß Anspruch 4, eine Wirtszelle gemäß Anspruch 5, ein Polypeptid gemäß Anspruch 6 oder 7 und/oder einen Antikörper gemäß einem der Ansprüche 8 bis 9, gegebenenfalls mit einem pharmazeutisch verträglichen Träger, umfasst.

11. Verwendung der pharmazeutischen Zusammensetzung nach Anspruch 10 zur Herstellung eines Arzneimittels zur Behandlung von Diabetes insipidus, Diabetes mellitus, Bluthochdruck, Magenulcera, Schilddrüsenerkrankungen und/oder Herzinsuffizienz.

12. Kit,
**dadurch gekennzeichnet, dass**
er ein Nukleinsäuremolekül gemäß einem der Ansprüche 1 bis 3, einen Vektor gemäß Anspruch 4, eine Wirtszelle gemäß Anspruch 5, ein Polypeptid gemäß Anspruch 6 oder 7, einen Antikörper gemäß einem der Ansprüche 8 bis 9, gegebenenfalls mit einem pharmazeutisch verträglichen Träger, und/oder die pharmazeutische Zusammensetzung gemäß Anspruch 10 umfasst.

13. erfahren zur Detektion einer AKAP-PKA-Interaktion umfassend die Schritte
a) Bereitstellung eines (i) ersten Vektors umfassend ein erstes Nukleinsäuremolekül gemäß einem der Ansprüche 1-3 und einen ersten Marker und eines (ii) zweiten Vektors umfassend ein zweites Nukleinsäuremolekül kodierend eine regulatorische Untereinheit einer Proteinkinase und einen zweiten Marker,
b) Einführen des ersten und des zweiten Vektors in eine Zelle, wobei die Zelle transfiziert wird und
c) Durchführen einer Fluoreszenzresonanzenergietransfer-(FRET)-Messung, wobei die AKAP-PKA-Interaktion detektiert wird, insbesondere die Interaktion zwischen AKAP und RIIα, RIIß, RIα und/oder RIß.

14. Verfahren zur Identifizierung von Inhibitoren der Interaktion von AKAP18 delta mit PKA, umfassend die Schritte nach Anspruch 13,
**dadurch gekennzeichnet, dass**
das Verfahren jeweils mit und ohne Zusatz des zu untersuchenden Inhibitors durchgeführt wird und einen Hinweis auf den AKAP- und/oder PKA-Inhibitor gibt.

15. Verfahren zur Identifizierung membranpermeabler Moleküle umfassend die Schritte nach Anspruch 13,
**dadurch gekennzeichnet, dass**
ein membranpermeables Molekül identifiziert wird, indem ein Konjugat aus dem zu untersuchenden membranpermeablen Molekül und einem membranpermeablen AKAP-PKA-Inhibitor hergestellt wird und die AKAP-PKA-Interaktion mit und ohne Konjugat oder das Molekül detektiert wird.

16. Verwendung eines Nukleinsäuremoleküls nach einem der Ansprüche 1 bis 3, eines Vektors nach Anspruch 4, einer Wirtszelle nach Anspruch 5, eines Polypeptids nach Anspruch 66 oder 7, einen Antikörper gemäß einem der Ansprüche 8 bis 9, einer pharmazeutischen Zusammensetzung nach Anspruch 10, eines Kits nach Anspruch 12 und/oder eines Verfahrens nach einem der Ansprüche 13 bis 15 zur Detektion einer AKAP-PKA-Interaktion oder eines AKAP-und/oder PKA-Inhibitors und/oder eines membranpermeablen Peptids.

## Claims

1. Isolated nucleic acid molecule selected from the group comprising
a) a nucleic acid molecule comprising a nucleotide sequence selected from the group consisting of the nucleotide sequence of Fig. 1A or its complementary nucleotide sequence,
b) a nucleic acid molecule, which hybridizes to a nucleotide sequence according to a) under stringent conditions and codes for a protein, which is locatable with AQP2 on intra-cellular vesicles in renal principle cells,
c) a nucleic acid molecule, which is degenerated to a nucleotide sequence according to a)as a consequence of the genetic code.

2. Nucleic acid molecule according to claim 1,
**characterized in that**
the nucleotide sequence stated in b) is at least 80%, preferably 90% homologous to a nucleotide sequence stated in a).

3. Nucleic acid molecule according to claim 1 or 2,
**characterized in that**
it is a genomic DNA, a cDNA and/or an RNA.

4. Vector comprising a nucleic acid molecule according to one of claims 1 to 3.

5. Host cell comprising the vector according to claim 4.

6. Polypeptide, coded by a nucleic acid molecule according to one of claims 1 to 3.

7. Polypeptide comprising the amino acid sequence of Fig. 1A.

8. Antibody, directed against the polypeptides according to claim 7, wherein the antibody binds AKAP18 delta specifically.

9. Antibody according to claim 8,
**characterized in that**
the antibody is an antibody fragment, in particular Fab, Fab', F(ab')₂, Fᵥ or a single chain antibody and/or an oligomeric, a reduced, an oxidized and/or a marked antibody.

10. Pharmaceutical composition,
**characterized in that**
it comprises a nucleic acid molecule according to one of claims 1 to 3, a vector according to claim 4, a host cell according to claim 5, a polypeptide according to claim 6 or 7 and/or an antibody according to one of claims 8 to 9, possibly with a pharmaceutically acceptable carrier.

11. Use of the pharmaceutical composition according to claim 10 for the production of a medicament for the treatment of diabetes insipidus, diabetes mellitus, hypertension, gastric ulcers, thyroid disorders and/or cardiac insufficiency.

12. Kit,
**characterized in that**
it comprises a nucleic acid molecule according to one of claims 1 to 3, a vector according to claim 4, a host cell according to claim 5, a polypeptide according to claim 6 or 7, an antibody according to one of claims 8 to 9, possibly with a pharmaceutically acceptable carrier and/or the pharmaceutical compound according to claim 10.

13. Method for detecting an AKAP-PKA interaction comprising the following steps:
a) provision of a (i) first vector comprising a first nucleic acid molecule according to one of claims 1 to 3 and a first marker and a (ii) second vector comprising a second nucleic acid molecule coding a regulatory subunit of a protein kinase and a second marker,
b) introduction of the first and second vector into a cell, wherein the cell is transfected, and
c) implementation of a fluorescence resonance energy transfer (FRET) measurement, wherein the AKAP-PKA interaction is detected, in particular the interaction between AKAP and RIIα, RIIβ, RIα and/or RIβ.

14. Method for the identification of inhibitors of the interaction of AKAP18 delta with PKA, comprising the steps according to claim 13,
**characterized in that**
the method is carried out both with and without the addition of the inhibitor to be examined and provides information on the AKAP and/or PKA inhibitor.

15. Method for the identification of membrane-permeable molecules comprising the steps according to claim 13,
**characterized in that**
a membrane permeable molecule is identified **in that** a conjugate is made of the membrane permeable molecule to be examined and a membrane permeable AKAP-PKA inhibitor and the AKAP-PKA interaction is detected with and without the conjugate or the molecule.

16. Use of a nucleic acid molecule according to one of claims 1 to 3, a vector according to claim 4, a host cell according to claim 5, a polypeptide according to claim 6 or 7, an antibody according to one of claims 8 to 9, a pharmaceutical compound according to claim 10, a kit according to claim 12 and/or a method according to one of claims 13 to 15 for the identification of an AKAP-PKA interaction or of an AKAP and/or PKA inhibitor or of a membrane permeable peptide.

## Revendications

1. Molécule d'acide nucléique isolée sélectionnée dans le groupe comprenant
a) une molécule d'acide nucléique comprenant une séquence nucléotidique sélectionnée dans le groupe comprenant la séquence nucléotidique de la fig. 1A ou sa séquence nucléotidique complémentaire,
b) une molécule d'acide nucléique qui, dans des conditions strictement déterminées, hybride avec une séquence nucléotidique selon a) et encode une protéine qui peut être localisée à l'aide d'AQP2 sur les vésicules intracellulaires dans des cellules rénales principales,
c) une molécule d'acide nucléique qui, suite au code génétique, est dégénérée en séquence nucléotidique selon a).

2. Molécule d'acide nucléique selon la revendication 1,
**caractérisée en ce que**
la séquence nucléotidique selon b) est homologue à au moins 80% et de préférence à 90% à une séquence nucléotidique selon a).

3. Molécule d'acide nucléique selon la revendication 1 ou 2,
**caractérisé en ce qu'**elle
est un ADN génomique, un cADN et/ou un ARN.

4. Vecteur comprenant une molécule d'acide nucléique selon l'une des revendications 1 à 3.

5. Cellule hôte comprenant le vecteur selon la revendication 4.

6. Polypeptide, encodé par une molécule d'acide nucléique selon l'une des revendications 1 à 3.

7. Polypeptide avec la séquence d'acide aminé selon la fig 1A.

8. Anticorps contre un polypeptide selon la revendication 7, l'anticorps liant spécifiquement AKAP18 delta.

9. Anticorps selon la revendication 8,
**caractérisé en ce que**
l'anticorps est un fragment d'anticorps, tel que notamment Fab, Fab', F(ab')₂, Fᵥ, ou bien un anticorps à chaîne unique et/ou un anticorps oligomère, réduit, oxydé et/ou un anticorps marqué.

10. Composition pharmaceutique,
**caractérisée en ce qu'**elle
englobe une molécule d'acide nucléique selon l'une des revendications 1 à 3, un vecteur selon la revendication 4, une cellule hôte selon la revendication 5, un polypeptide selon la revendication 6 ou 7 et/ou un anticorps selon l'une des revendications 8 à 9, le cas échéant, avec un porteur pharmaceutiquement compatible.

11. Utilisation de la composition pharmaceutique selon la revendication 10 pour la production d'un médicament pour le traitement du diabète insipidus, du diabète mellitus, de l'hypertension, d'ulcères gastriques, de maladies thyroïdiennes et/ou d'insuffisance cardiaque.

12. Kit,
**caractérisé en ce qu'**il
comprend une molécule d'acide nucléique selon l'une des revendications 1 à 3, un vecteur selon la revendication 4, une cellule hôte selon la revendication 5, un polypeptide selon la revendication 6 ou 7, un anticorps selon l'une des revendications 8 à 9, le cas échéant, avec un porteur pharmaceutiquement compatible, et/ou la composition pharmaceutique selon la revendication 10.

13. Procédé pour la détection d'une interaction AKAP-PKA comprenant les étapes suivantes :
a) mise à disposition d'un (i) premier vecteur comprenant une première molécule d'acide nucléique selon l'une des revendications 1 à 3 et un premier marqueur et d'un (ii) deuxième vecteur comprenant une deuxième molécule d'acide nucléique encodant une sous-unité régulatrice d'une protéine kinase et un deuxième marqueur,
b) introduction du premier et du deuxième vecteur dans une cellule, cette cellule étant transfectée et
c) réalisation d'une mesure de transfert d'énergie de fluorescence (FRET), l'interaction AKAP-PKA étant détectée et notamment l'interaction entre AKAP et RIIα, RIIβ, RIα et/ou RIβ.

14. Procédé pour l'identification d'inhibiteurs de l'interaction entre AKAP18 delta et PKA, englobant les étapes selon la revendication 13,
**caractérisé en ce que**
le procédé est réalisé à chaque fois avec et sans l'ajout de l'inhibiteur à examiner et informe sur l'inhibiteur AKAP et/ou PKA.

15. Procédé pour l'identification de molécules à perméabilité membranaire englobant les étapes selon la revendication 13,
**caractérisé en ce qu'**une
molécule à perméabilité membranaire est identifiée en réalisant un système conjugué à partir de la molécule à perméabilité membranaire à examiner et d'un inhibiteur AKAP-PKA à perméabilité membranaire et que l'interaction AKAP-PKA est détectée avec ou sans le système conjugué et avec ou sans la molécule.

16. Utilisation d'une molécule d'acide nucléique selon l'une des revendications 1 à 3, d'un vecteur selon la revendication 4, d'une cellule hôte selon la revendication 5, d'un polypeptide selon la revendication 6 ou 7, d'un anticorps selon l'une des revendications 8 à 9, d'une composition pharmaceutique selon la revendication 10, d'un kit selon la revendication 12 et/ou d'un procédé selon l'une des revendications 13 à 15 pour la détection d'une interaction AKAP-PKA ou d'un inhibiteur AKAP et/ou PKA et/ou d'un peptide à perméabilité membranaire.
